(19) Europäisches Patentamt
European Patent Office
Office européen des brevets

(11) Numéro de publication: **0 017 536**
**B1**

# (12) FASCICULE DE BREVET EUROPEEN

(45) Date de publication du fascicule du brevet: **23.03.83**

(21) Numéro de dépôt: **80400356.4**

(22) Date de dépôt: **18.03.80**

(51) Int. Cl.³: **C 07 C 103/52,**
A 61 K 37/02,
C 07 C 149/437

## (54) Procédé de préparation de la somatostatine.

(30) Priorité: **23.03.79 FR 7907430**

(43) Date de publication de la demande:
**15.10.80 Bulletin 80/21**

(45) Mention de la délivrance du brevet:
**23.03.83 Bulletin 83/12**

(84) Etats contractants désignés:
**CH DE GB IT SE**

(56) Documents cités:
**FR - A - 1 396 655**
**FR - A - 2 361 347**
**US - A - 3 933 784**
**US - A - 4 100 153**

**MIKLOS BODANSZKY: PEPTIDE SYNTHESIS, Interscience publishers, pages 21—32; Easily removable protecting groups and their removal**

(73) Titulaire: **SANOFI, société anonyme**
**40, Avenue George V**
**F-75008 Paris (FR)**

(72) Inventeur: **Diaz, Joseph**
**19 rue du Pradas**
**F-34470 Perols (FR)**
Inventeur: **Guegan, Rémy**
**Chemin du Thym**
**F-34170 Castelnau le Lez (FR)**

(74) Mandataire: **de Haas, Michel et al,**
**Cabinet Beau de Loménie 55 rue d'Amsterdam**
**F-75008 Paris (FR)**

**EP 0 017 536 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Procédé de préparation de la somatostatine

La somatostatine a été isolée par l'équipe de Guillemin à partir d'extraits d'hypothalamus de moutons [comptes de l'Académie des Sciences de Paris, *275,* 2913 (1972)]. La même équipe en a indiqué la structure qui correspond à un tétradécapeptide cyclique avec une liaison disulfure réunissant les groupes cystéine en positions 3 et 14:

H. Ala—Gly—Cys—Lys—Asn—Phe—Phe—Trp—Lys—Thr—Phe—Thr—Ser—Cys—OH
1 2 3 4 5 6 7 8 9 10 11 12 13 14

Dans cette formule, tous les aminoacides, à l'exception de la glycine, sont dans la configuration L et les abréviations utilisées pour les aminoacides sont celles recommandées par la Commission IUPAC—IUB de nomenclature biochimique [Journal of Biological Chemistry, *247,* 977 (1972)].

La somatostatine possède une grande variété d'effets biologiques, en particulier au niveau de l'inhibition de la sécrétion de l'hormone de croissance et de l'inhibition des sécrétions d'insuline et de glucagon, ainsi que de l'inhibition de certaines sécrétions digestives.

Par suite, la somatostatine s'est révélée particulièrement intéressante en tant qu'agent thérapeutique, en particulier pour le traitement de l'acromégalie, du diabète, ainsi qu'en gastro-entérologie. En conséquence, il s'est révélé néccessaire de produire des quantités suffisantes de somatostatine très pure pour poursuivre les investigations cliniques du produit.

Différentes synthèses de la somatostatine ont déjà été décrites.

Certaines méthodes proposées utilisent la synthèse en phase solide (brevets des Etats-Unis d'Amérique n° 3 862 925 et n° 3 904 594 en particulier), alors que d'autres ont lieu en phase liquide selon la technique des fragments [Helvetica Chemica Acta, *57,* 730 (1974), ou Biochemical Biophysical Research Communication *54,* 234 (1973), etc.). Enfin, dernièrement, un procédé "Stepwise" a été mentionné [International Journal of Peptides and Proteines Research, *11,* 329 (1978)].

Toutefois, ces différents procédés sont tous inadaptés à une production industrielle à grande échelle de la somatostatine.

Ainsi, il est connu que les procédés de synthèse des peptides en phase solide ne sont pas transposables à l'échelle industrielle, en particulier à cause des difficultés rencontrées pour scinder le polypeptide de son support après la phase d'élongation. La scission nécessite des moyens énergiques et s'accompagne d'une dégradation importante conduisant à des rendements faibles et nécessitant des purifications subséquentes longues et difficiles.

De même, les différentes synthèses en phase liquide connues à ce jour ne conduisent à la somatostatine qu'avec des rendements très faibles et nécessitent des purifications laborieuses et nombreuses pendant les phases intermédiaires.

Le procédé suivant l'invention a pour objet de surmonter toutes ces difficultés et de permettre l'obtention de quantités importantes de somatostatine de haute pureté. Ce procédé est un procédé étape par étape, dit "procédé Stepwise" à partir du C terminal, caractérisé en ce qu'on utilise, dès le début de la synthèse, un groupement protecteur de la fonction acide de la cystéine en position 14, qui insolubilise la molécule dans l'eau et les solvants organiques usuels à l'exception du diméthylformamide et du diméthylsulfoxyde. Les réactions de couplage peptidique sont effectuées en solution dans le diméthylformamide et le produit de la réaction est isolé soit par évaporation du diméthylformamide, soit par précipitation par addition d'un deuxième solvant qui l'insolubilise. Le peptide brut ainsi obtenu est purifié par des lavages en phase solide à l'aide de solvants appropriés qui dissolvent les impuretés.

Cette technique présente donc au niveau des lavages les mêmes avantages que la synthèse en phase solide sans en présenter les inconvénients (risque de réaction incomplète au niveau du couplage en particulier).

Les rendements obtenus à chaque étape sont excellents et se situent entre 90 et 98% de la théorie.

Dans le procédé utilisé, les fonctions hydroxyle de la sérine et des deux molécules de thréonine n'ont pas besoin d'être protégées, ce qui représente un avantage important car ces protections sont toujours difficiles à réaliser. Les protections des chaînes latérales des deux molécules de lysine (fonction amine en $\omega$) et de la fonction acide terminale de la cystéine en position 14 ont été choisies de façon à résister aux traitements acides nécessaires pendant les phases d'élongation pour libérer les protections temporaires des fonctions $\alpha$-aminées.

Ce sont donc des groupes protecteurs clivables seulement en milieu basique fort, à savoir le groupe méthanesulfonyléthyloxycarbonyle (MSC) pour les amines en $\omega$ des molécules de lysine et le groupe phénylazobenzylsulfonyléthyloxy (OPSE) pour la fonction acide de la cystéine en position 14. C'est ce dernier groupement qui confère en particulier, dès le départ, leur caractère d'insolubilité aux différents peptides intermédiaires obtenus au cours de la phase d'élongation.

Enfin, les deux groupements thiol des molécules de cystéine en positions 3 et 14 ont été protégés

par un groupement qui résiste à la fois aux conditions acides et basiques, afin de pouvoir être conservé jusqu'à la fin de la synthèse. Le groupement protecteur ainsi choisi est le groupe acétamidométhyle (Acm).

Le premier élément de la synthèse est constitué par le N-t-butoxycarbonyl S-acétamidométhyl L-cystéinate de p-phénylazobenzylsulfonyléthyle.

Ce composé est nouveau et s'obtient par condensation de la N-t-butoxycarbonyl S-acétamidométhylcystéine su le p-phénylazobenzylsulfonyléthanol en présence d'un agent de déshydratation tel que le dicyclohexylcarbodiimide.

Ce composé est obtenu avec un excellent rendement; il est peu soluble dans l'eau et présente une coloration jaune orangé facilitant les suivis chromatographiques. Enfin, le groupement protecteur de l'amine tertiobutyloxycarbonyle peut être facilement éliminé à l'aide d'un acide fort, tel que l'acide trifluoracétique ou l'acide formique contenant 1% d'acide chlorhydrique.

A partir de ce produit de départ, on procède à l'élongation de la chaîne polypeptidique par couplage de l'acide aminé suivant convenablement protégé, si nécessaire.

Chaque phase de couplage est suivie d'une opération sélective de libération de l'amine qui va entrer en réaction lors de la création de la liaison peptidique suivante. On opère en milieu acide fort tel que l'acide trifluoracétique ou l'acide formique à 1% d'acide chlorhydrique.

Les groupes protecteurs temporaires des fonctions aminées en $\alpha$, clivables en milieu acide, sont choisis dans la famille des carbamates. Ce sont soit le groupe N-tertiobutoxycarbonyle (Boc), soit le groupe N-$\alpha$-biphénylisopropyloxycarbonyl (B Poc).

Dès que le L-tryptophane est en place (position 8), il devient indispensable, au moment des phases de clivage des groupements protecteurs intermédiaires, d'utiliser des adjuvants chimiques qui protégent le noyau indole de réactions parasites spécifiques de cet hétérocycle.

C'est ainsi que l'addition d'anisole dans le milieu prévient les réactions radicalaires, tandis que l'utilisation de thiols tels que l'éthanedithiol ou le mercapto-éthanol évite les oxydations parasites de cet hétérocycle.

D'un façon générale, on préfère utiliser dans les phases de couplage les $\alpha$-aminoacides convenablement protégés sous forme d'esters activés.

Le solvant de réaction est choisi de façon à maintenir en solution à la fois les réactifs et les produits de la réaction. Les solvants de choix, compte tenu de caractère d'insolubilité des polypeptides obtenus, sont le diméthylformamide et le diméthylsulfoxyde.

L'utilisation d'un activateur de couplage, tel que l'hydroxybenzotriazole, a un effet bénéfique sur la vitesse de réaction tout en minimisant les risques de racémisation.

Enfin, il est avantageux d'introduire les deux derniers aminoacides ensemble sous la forme de dipeptide activé.

L'ensemble des opérations est représenté dans les schémas de synthèse I et II ci-après.

Les rendements sont excellents à tous les niveaux et la pureté des produits est satisfaisante. Les contrôles sont effectués par chromatographie en couche mince sur plaques de silicagel dans différents milieux d'élution.

SCHEMA I

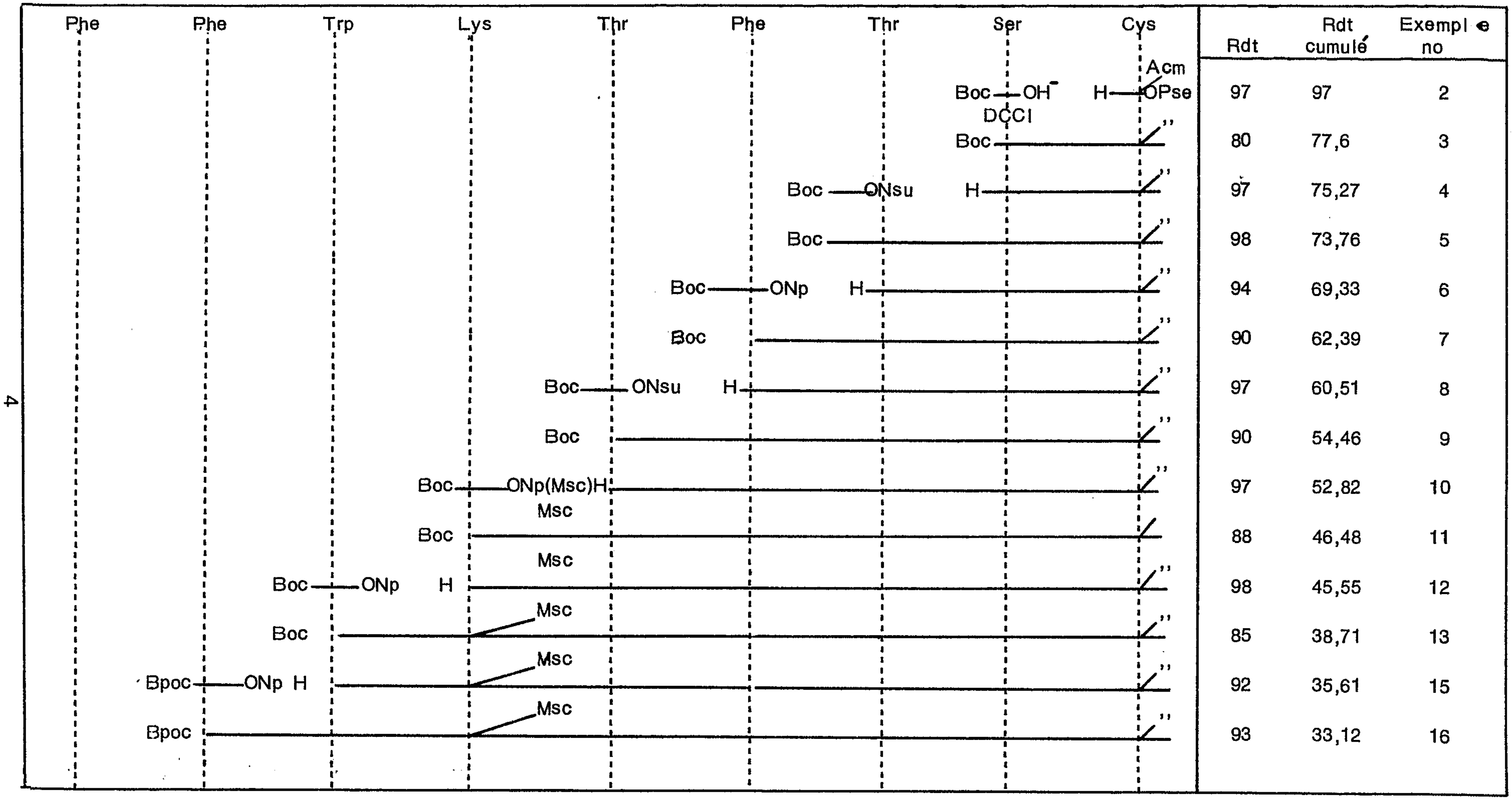


| Rdt | Rdt cumulé | Exemple no |
|---|---|---|
| 97 | 97 | 2 |
| 80 | 77,6 | 3 |
| 97 | 75,27 | 4 |
| 98 | 73,76 | 5 |
| 94 | 69,33 | 6 |
| 90 | 62,39 | 7 |
| 97 | 60,51 | 8 |
| 90 | 54,46 | 9 |
| 97 | 52,82 | 10 |
| 88 | 46,48 | 11 |
| 98 | 45,55 | 12 |
| 85 | 38,71 | 13 |
| 92 | 35,61 | 15 |
| 93 | 33,12 | 16 |

SCHEMA II

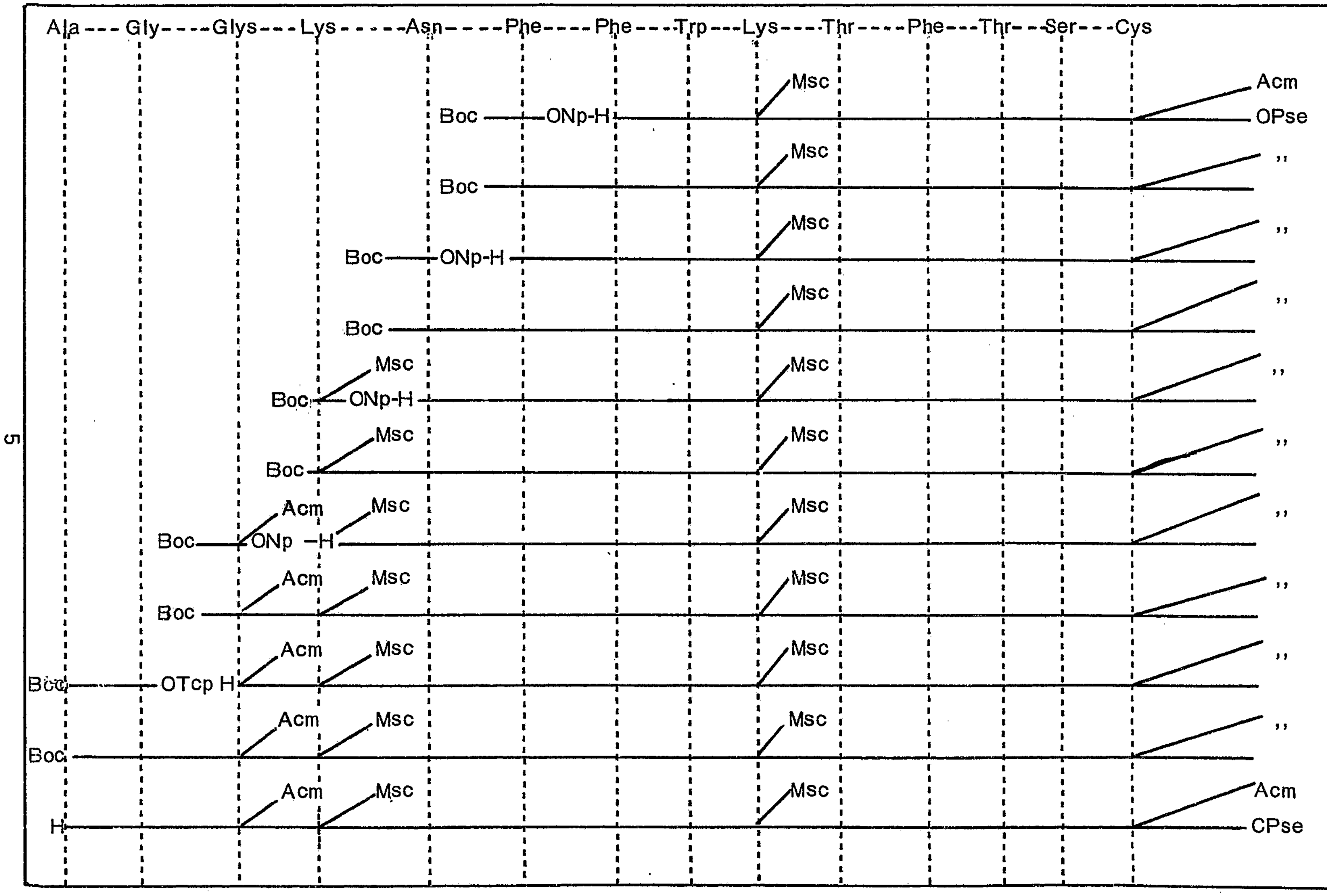


| Rdt | Rdt cumulé | Exemple n° |
|---|---|---|
| 97 | 32,13 | 17 |
| 95 | 30,52 | 18 |
| 100 | 30,52 | 20 |
| 87 | 26,55 | 21 |
| 100 | 26,55 | 22 |
| 92 | 24,43 | 23 |
| 100 | 24,43 | 24 |
| 93 | 22,72 | 25 |
| 100 | 22,72 | 26 |
| 93 | 21,58 | 27 |
| 100 | 21,58 | 28 |

Une variante du procédé consiste à effecteur séparément la synthèse des heptapeptides 1 à 7 et 8 à 14 également selon le même principe du Stepwise. Les deux heptapeptides sont ensuite couplés par un agent chimique convenable en tétradécapeptide.

Cette variante présente l'avantage de ne pas soumettre le tryptophane, aminoacide particulièrement sensible à l'oxydation et aux réactions radicalaires, à une suite de réactions pour effectuer les phases de déprotection subséquentes à chaque couplage des aminoacides 1 à 7.

L'heptapeptide 8 à 14:

```
             MSC                     Acm
              |                       |
H—Trp—Lys—Thr—Phe—Thr—Ser—Cys—O Pse
```

est indentique à l'heptapeptide intermédiaire de la stratégie Stepwise complète.

L'heptapeptide 1 à 7:

```
                  Acm MSC
                   |   |
Boc—Ala—Gly—Cys—Lys—Asn—Phe—Phe—OH
```

est obtenu par un procédé particulier dans lequel l'acide terminal n'est pas protégé pendant la phase d'élongation, ce qui permet d'effectuer les réactions de couplage en phase liquide et les lavages en phase solide.

On part du dipeptide connu H—Phe—Phe—$OCH_3$, sur lequel on fait réagir l'ester orthonitrophénolique de l'orthonitrophénylsulfénamino asparagine dans le diméthylformamide. Le tripeptide est isolé par précipitation avec de l'éther et purifié par des lavages en phase solide. Ce tripeptide est entièrement déprotégé par un traitement basique (saponification de l'ester), puis par acidolyse qui libère la fonction amine de la lysine.

Ce tripeptide H—Asn—Phe—Phe—OH est utilisé sous sa forme acide libre terminal pour la suite des opérations.

Chaque acide aminé est introduit sous forme d'ester activé et la réaction est conduite en présence d'un accélérateur tel que l'hydroxybenzotriazole et en présence d'une base organique non racémisante telle que la N-éthylmorpholine.

L'ensemble des différentes étapes est rassemblé sur le schéma de synthèse III ci-après.

Le couplage entre les deux heptapeptides peut être effectué dans le diméthylformamide en présence d'un agent de couplage tel que le dicyclohexylcarbodiimide, ou bien à l'aide d'un activateur de la fonction carboxylique comme l'hexafluorophosphate de benzotriazolyloxy-(tridiméthylamino)-phosphonium.

Le tétradécapeptide obtenu par cette voie est absoluement identique à celui obtenu par le procédé Stepwise complet.

Quelle que soit la variante utilisée, on aboutit ainsi au tétradécapeptide protégé:

```
         Acm Msc                     Msc                     Acm
          |   |                       |                       |
Boc—Ala—Gly—Cys—Lys—Asn—Phe—Phe—Trp—Lys—Thr—Phe—Thr—Ser—Cys—OPse
```

Un traitement en milieu acide fort tel que l'acide trifluoroacétique permet de libérer la fonction amine de l'alanine en position 1. Par action d'une base forte telle que la baryte, pendant un temps relativement court (15 à 30 min) et à température ambiante sur une solution du produit dans le diméthylformamide, on élimine à la fois les groupes protecteurs des chaînes latérales des deux lysines et l'ester sur la cystéine en position 14.

On est ansi conduit à la di-S-acétamidométhyl-3,14-dihydrosomatostatine qui est soumise à une purification avant d'aborder les étapes finales.

SCHEMA III

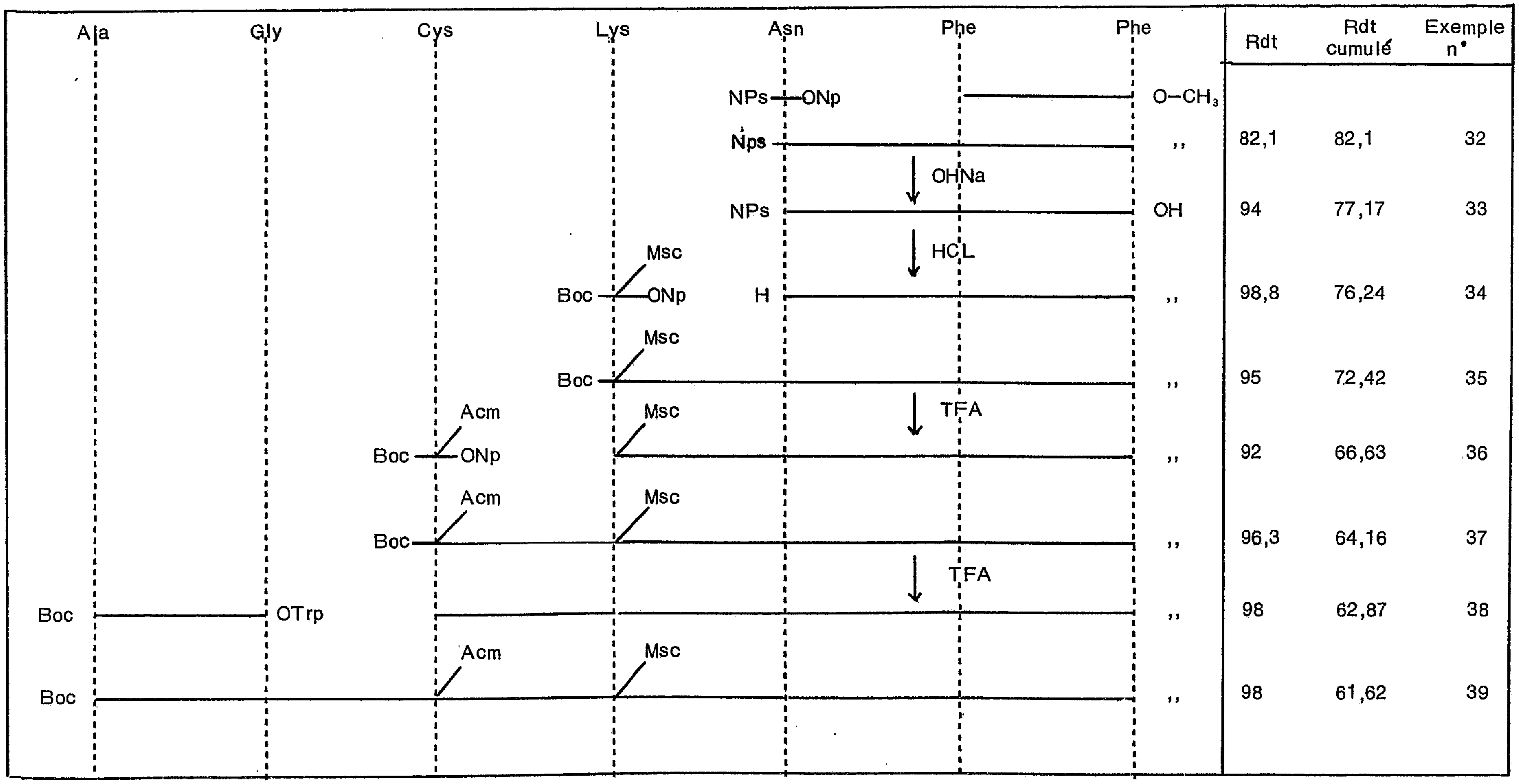


| Rdt | Rdt cumulé | Exemple n° |
|---|---|---|
| 82,1 | 82,1 | 32 |
| 94 | 77,17 | 33 |
| 98,8 | 76,24 | 34 |
| 95 | 72,42 | 35 |
| 92 | 66,63 | 36 |
| 96,3 | 64,16 | 37 |
| 98 | 62,87 | 38 |
| 98 | 61,62 | 39 |

On peut utiliser, pour la purification, différentes méthodes telles que la distribution à contre-courant, la chromatographie de partition sur gel, la chormatographie sur échangeurs d'ions, etc. Sur des quantités importantes, la distribution à contrecourant s'est avérée la méthode de choix et permet d'obtenir un produit de pureté supérieure ou égale à 98%. La libération de la fonction thiol des deux cystéines s'obtient facilement selon des méthodes connues, à savoir par traitement par un sel de métal lourd tel que le mercure ou l'argent. Il est possible, éventuellement, d'isoler le sel de la dihydrosomatostatine avec le métal. Celui-ci peut être conservé, si besoin est, et sans altération, pendant plusieurs semaines à +4°C à l'abri de la lumière.

Un traitement par l'hydrogène sulfuré permet d'éliminer le métal sous forme de sulfure insoluble facile à éliminer.

La solution ainsi obtenue contient la dihydrosomatostatine qu'il est possible d'isoler par évaporation à température ambiante.

Le plus souvent, la solution est soumise au pontage final en disulfure en utilisant des techniques connues d'oxydation déjà utilisées pour la somatostatine elle-même ou pour d'autres polypeptides tels que la vasopressine ou l'oxytocine. On peut, par exemple, utiliser l'oxygène de l'air, ou encore le ferricyanure de potassium. On a de toute, façon intérêt à opérer en solution très diluée, de façon à minimiser au maximum les réactions de polymérisation.

Dans le schéma IV ci-après, l'ensemble des opérations à partir du tétradécapeptide protégé est résumé. La somatostatine brute ainsi obtenue ne nécessite qu'une chromatographie de perméation sur gel de Sephadex G 25 pour obtenir un produit de haute pureté dont le titre est supérieur à 98% par détermination en HPLC.

Le procédé ainsi décrit permet de préparer des quantités importantes de somatostatine en n'effectuant des purifications poussées qu'à deux niveaux:

— di-S-acétamidométhyl-3,14 dihydrosomatostatine,
— somatostatine elle-même.

Dans toutes les autres étapes, les phases de purification sont limitées à des opérations de lavage en phase hétérogène.

SCHEMA IV

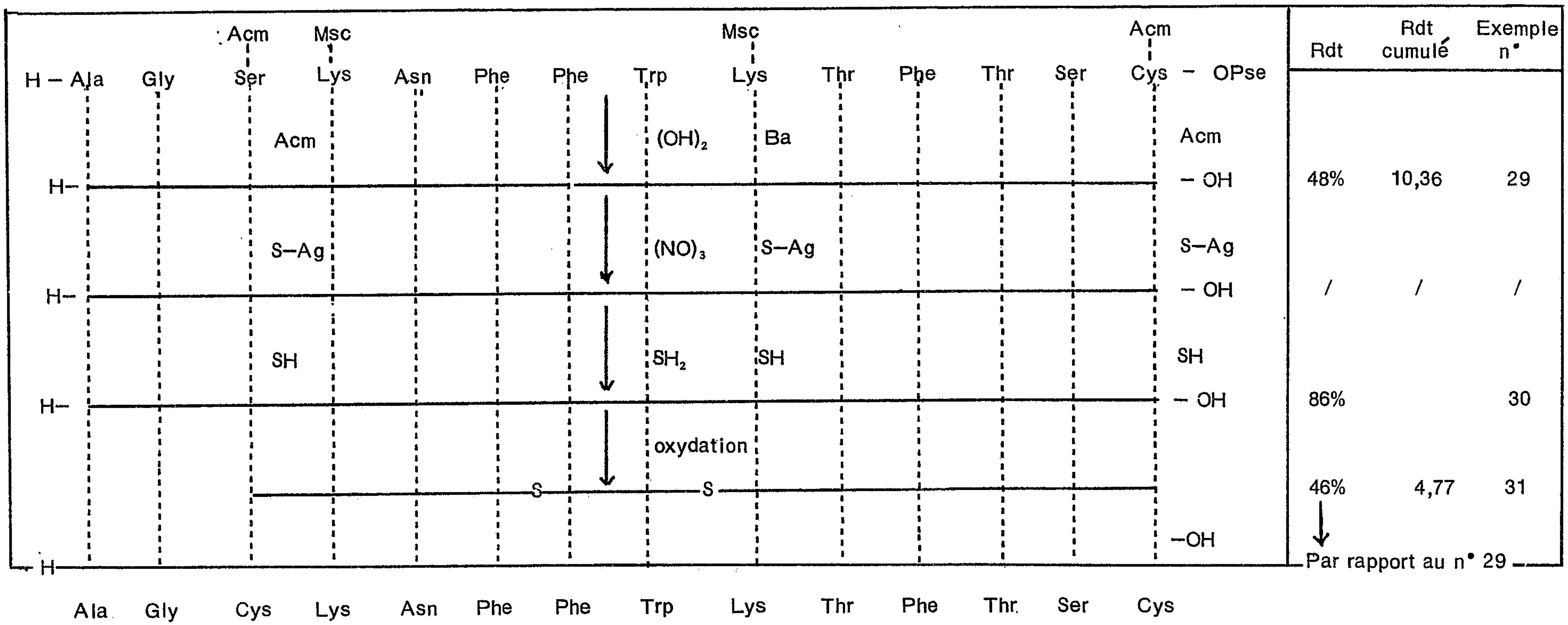


| Rdt | Rdt cumulé | Exemple n° |
|---|---|---|
| 48% | 10,36 | 29 |
| / | / | / |
| 86% | | 30 |
| 46% | 4,77 | 31 |

↓ Par rapport au n° 29

Les caractéristiques et avantages du procédé selon l'invention seront mieux compris à la lecture des exemples suivants.

Dans les exemples qui vont suivre, les abréviations suivantes seront utilisées.

Groupes protecteurs:

| | | |
|---|---|---|
| Acm | = | acétamidométhyle |
| Boc | = | t-butoxycarbonyle |
| OPse | = | phénylazobenzylsulfonyléthoxy |
| ONsu | = | N-succinimidoxy |
| ONp | = | nitro-4 phénoxy |
| OTcp | = | trichloro-2,4,5 phénoxy |
| Nps | = | o-nitrophènylsulfényle |
| Msc | = | (méthylsulfonyl)-2 éthoxycarbonyle |

Eluants utilisés en chromatographie sur couche mince de silice:

Composition en volume.

| | |
|---|---|
| *Truth* | : chloroforme : 95 — méthanol : 5 — acide acétique : 3 |
| *BEW 1* | : butanol-2 : 72 — acide acétique : 7 — eau : 21 |
| *BPEW 1* | : butanol-1 : 50 — acide acétique : 12 — pyridine : 12 — eau : 25 |
| *EBPEW* | : acétate d'éthyle : 42 — butanol-1 : 24 — pyridine : 21 — acide acétique : 6 — eau : 10 |
| *EPAW 2* | : Acétate d'éthyle : 70 — pyridine : 16 — acide formique : 8 — eau : 6 |
| *EPAW 1* | : acétate d'éthyle : 63 — pyridine : 21 — acide formique : 10 — eau : 6 |

Abréviations diverses:

| | |
|---|---|
| Fc | : point de fusion en tube capillaire |
| DMF | : diméthylformamide |
| DCCI | : dicyclohexylcarbodiimide |
| AcOH | : acide acétique |
| TFA | : acide trifluoroacétique |

## Exemple 1

(L)-N-t-butoxycarbonyl S-acétamidométhylcystéinate de p-phénylazobenzylsulfonyléthyle

Acm
|
Boc—Cys—OPse

a) *N-hydroxyméthylacétamide*

250 g d'acétamide, 150 g polyoxyméthylène et 2,5 g de carbonate de potassium sont pulvérisés ensemble et fondus à 100°C. Après 1 h à cette température et refroidissement vers 50—60°C, on ajoute 0,5 ml de potasse aqueuse 12 N et abandonne au réfrigérateur une nuit. Le solide pris en masse est repris dans le minimum d'acétone pure (environ 200 ml) pour le dissoudre à température ambiante. Le produit cristallise par refroidissement quelques heures à —30°C, il est alors filtré rapidement et séché.

On obtient ainsi 170 g; Fc 45—50°C.

Un deuxième jet d'environ 50 g peut être obtenu en concentrant le solvant.

b) *(L)-S-acétamidométhylcystéine chlorhydrate*

H—Cys (Acm)—OH, Cl H

75 g (0,84 mole) d'acétamido méthanol en solution dans 300 ml de dioxanne sont ajoutés goutte à goutte à température ambiante à un mélange bien agité de 88 g (0,5 mole) de chlorhydrate de L-cystéine monohydrate dans 1 250 ml de dioxanne, 22 ml d'acide chlorhydrique concentré et 40 ml d'eau. L'agitation est poursuivie 30 min, l'huile formée est décantée et le solvant qu'elle contient est évaporé en évaporateur rotatif à 30°C. Le résidu est repris par de l'éthanol qui est évaporé à son tour pour déshydrater le produit; il cristallise progressivement et est filtré sous vide, lavé à l'isopropanol, puis à l'éther et séché sous vide.

On obtient ainsi 90 g (rendement 80%); Fc 162—165°C.

$[\alpha]_D^{25} = -27,5°$ (C = 1, eau).

Un échantillon recristallisé dans le mélange isopropanol-eau 80/20 (volume/volume) fournit un produit de Fc 166—169°C.

$[\alpha]_D^{25} = -27°$ (C = 1, eau).

c) *(L)-N-t-butoxycarbonyl S-acétamidométhylcystéine*

Acm
|
Boc—Cys—OH

18,2 g (0,08 mole) de chlorhydrate d'acétamidométhylcystéine en solution dans 130 ml d'un mélange dioxanne-eau 50/50 (volume/volume) sont portés à 30—35°C au ba in-marie et le pH ajusté à 10,0 par NaOH 4N. On ajoute alors 12,3 g (11,2 ml, 0,088 mole) d'azidoformiate de t-butyle et le pH maintenu à 10,0 par addition de NaOH 4N. La réaction est complète dans un temps compris entre 18 et 30 h (fonction de la température). On ajoute alors 70 ml d'eau et lave par deux fois 100 ml d'éther pour éliminer l'excès de réactif. La phase aqueuse refroidie à 0+5°C est acidifiée en présence d'acétate d'éthyle à pH 2,5 par une solution 4N de bisulfate de potassium, puis la phase aqueuse est extraite trois fois par l'acétate d'éthyle. Les extraits organiques réunis sont lavés par une solution saturée de chlorure de sodium (deux fois), séchés et évaporés sous vide partiel laissant une huile qui cristallise peu à peu en présence d'éther anhydre.

19,5 g (83%); Fc 112—114°C.
$[\alpha]_D^{25} = -29,5°$ (C = 1, $CH_3OH$).
$[\alpha]_D^{25} = -33,6°$ (C = 1, $H_2O$).

d) *(L) N-t-butoxycarbonyl S-acétamidométhylcystéinate de p-phénylazobenzylsulfonyléthyle*

Acm
|
Boc—Cys—OPse

Acm
|
32 g (0,110 mole) de Boc—Cys—OH

sont dissous dans 350 ml de pyridine pure suivis de 30,4 g (0,100 mole) de PseOH. Lorsque tout est homogène, la solution est refroidie à 0°C et traitée progressivement par 21,6 g (0,105 mole) de dicyclohexylcarbodiimide en solution dans 40 ml de pyridine pure. La solution est agitée une nuit au réfrigérateùr.

Un contrôle CCM/AcOET—$CH_3OH$: 96/4 (volume/volume) permet de vérifier la conversion totale de PseOH (Rf ~ 0,50—0,55) en produit attendu (Rf ~ 0,42—0,50). Si elle n'est pas complète, rajouter un peu de

Acm
|
Boc—Cys—OH

en excès car l'élimination du PseOH résiduel est difficile.

L'isolement du produit se fait de la manière suivante: la dicyclohexylurée (DCU) est filtrée, la pyridine évaporée sous vide à 30°C, le résidu repris par de l'acétate d'éthyle et la DCU filtrée encore, puis la solution lavée successivement par une solution de bisulfatesulfate de potassium (pH=2,0), de l'eau, une solution de bicarbonate de sodium à 5% dans l'eau, puis à l'eau et séchée sur sulfate de magnésium. Après filtration, on évapore le solvant, la gomme rouge obtenue se solidifie progressivement par contact avec 400 ml d'éther anhydre. Le gel obtenu est pulvérisé et filtré, rincé à l'éther anhydre et séché.

Poids: 53,3 g; rendement 92%; Fc 75—77°C.
$[\alpha]_D^{25} = -16°$ (C = 1, AcOH).
$[\alpha]_D^{25} = -24°$ (C = 1, $CH_3OH$).
CCM/AcO Et — MeOH Rf = 0,50; $BEW_1$ Rf = 0,69.
Truth Rf = 0,50; $BPEW_1$ Rf = 0,77.

Exemple 2

(L)-S-acétamidométhylcystéinate de p-phénylazobenzylsulfonyléthyle, chlorhydrate

Acm
|
H—Cys—OPse, HCl

Acm
|
5,78 g de Boc—Cys—OPse

sont ajoutés rapidement (1 à 2 min) à une solution de 1 ml d'acide chlorhydrique concentré dans 100 ml d'acide formique (98% pur) à température ambiante.

Après 10 min à cette température, la réaction est terminée. Le solvant est évaporé sous vide le plus rapidement possible à température ⩽30°C. Le résidu huileux repris dans 15 ml de méthanol est versé goutte à goutte dans 150 ml d'éther anhydre agité à 0°C. Le produit est filtré, rincé à l'éther anhydre et séché en présence d'anyhdride phosphorique sous vide. On obtient ainsi 5,0 g (97%) d'un chlorhydrate de Fc 160—162°C (décomposition).

CCM/$BEW_1$ Rf = 0,36 |
$BPEW_1$ Rf = 0,62 | produit unique

Exemple 3

N-t-butoxycarbonyl L-séryl S-acétamidométhyl L-cystéine p-phénylazobenzylsulfonyl-éthylester.

Acm
|
Boc—Ser—Cys—OPse

5,15 g (0,01 mole) de chlorhydrate de S-acétamidométhylcystéine sont traités en solution dans 60 ml de DMF pur anhydre par une solution de 1,15 g de N-éthylmorpholine, puis refroidis à −10°C et traités alors par 2,05 g de N-butoxycarbonyl L-sérine (ajoutés rapidement) puis goutte à goutte en 5—10 min par une solution de 2,47 g de DCCI et 2,70 g d'hydroxybenzotriazole dans 20 ml de DMF pur.

Après 1 h à −10°C, on laisse la température remonter progressivement à la température ambiante (20°C) en évitant tout échauffement.

La réaction est complète en 6 h. Le DMF est alors évaporé sous haut vide à 30—35°C et le résidu repris dans 200 ml d'acétate d'éthyle. La dicyclohexylurée est filtrée et on procède aux lavages suivants: sulfate et bisulfate de potassium aqueux, eau, bicarbonate de sodium aqueux à 5%, eau, chaque lavage étant répété deux ou trois fois.

Après séchage sur sulfate de magnésium à température ambiante le solvant est concentré à environ 50 ml. Le produit précipite en gel par refroidissement une nuit à 0°C. Il est filtré, rincé par un peu d'acétate d'éthyle, puis à l'éther anhydre et séché sous vide sans chauffer.

Rendement: 5,3 g (80%); Fc 111—113°C.

CCM/$BEW_1$ Rf = 0,63 |
$BPEW_1$ Rf = 0,70 | Révélateur Reindel-Hoppe
$EPAW_2$ Rf = 0,74 |

Dans ces divers systèmes, le produit renferme un peu de decyclohexylurée (Rf 0,80 à 0,86) qui s'éliminera au stade suivant.

$[\alpha]_D^{25} = -25,5$ (C = 1, $CH_3OH$).

Exemple 4

L-séryl S-acétamidométhyl L-cystéinate de p-phénylazobenzylsulfonyléthyle, trifluoracétate

Acm
|
H—Ser—Cys—OPse, $CF_3COOH$

Acm
|
38,5 g de Boc—Ser—Cys—OPse

sont ajoutés rapidement à 300 ml d'acide trifluoracétique préalablement refroidis vers 0°C (la température monte vers 18°C sur de telles quantités). Après 3 à 5 min, l'acide est évaporé à 20°C sous 15 mm. Le résidu est repris par de l'éther anhydre au contact duquel le produit se solidifie. Il est filtré et rincé à l'éther anhydre plusieurs fois et séché.

Rendement: 38,5 g (97%).

CCM/$EPAW_2$ Rf = 0,30
$BPEW_1$ Rf = 0,60
EBPEW Rf = 0,50

Exemple 5

N-t-butoxycarbonyl L-thréonyl L-séryl S-acétamidométhyl L-cystéine, p-phénylazobenzylsulfonylester

Acm
|
Boc—Thr—Ser—Cys—OPse

Acm
|
6,80 g de trifluoracétate de H—Ser—Cys—OPse

sont dissous dans 100 ml de DMF pur. Tout en refroidissant au bain de glace, on ajoute successivement 2,52 ml de N-éthylmorpholine et 3,79 g de Boc—Thr—ONSu. Le milieu réactionnel est conservé à température ambiante pendant 2 h. L'isolement se fait par évaporation du DMF à 30°C et reprise par la glace. On filtre sous vide puis lave avec de l'eau une solution bisulfate-sulfate de potassium (pH=2), avec de l'eau, du bicarbonate de sodium aqueux à 5% et enfin avec de l'eau deux fois. Après séchage à l'air, puis sur anhydride phosphorique sous vide, on obtient:

7,5 g (98%); Fc = 136—138°C.

CCM/EPAW$_2$ Rf = 0,70
BEW$_1$ Rf = 0,69
EBPEW Rf = 0,86

Exemple 6

L-thréonyl L-séryl S-acétamidométhyl L-cystéinate de p-phénylazobenzylsulfonyléthyle, trifluoracétate

Acm
|
$CF_3CCOH$, H—Thr—Ser—Cys—OPse

Acm
|
7,67 g (0,01 mole) de Boc—Thr—Ser—Cys—OPse

sont ajoutés à 60 ml d'acide trifluoracétique refroidis au bain de glace, sous agitation. Le bain froid est ôté et la solution conservée pendant 5 min avant d'évaporer l'acide trifluoracétique. Le résidu ainsi obtenu est repris à l'éther en présence duquel il est trituré, filtré et lavé abondamment, puis séché.

On obtient 7,34 g du trifluoracétate (94%).

CCM/EPAW$_2$ Rf = 0,30, produit pur
EPAW$_1$ Rf = 0,52, produit pur

Exemple 7

N-t-butoxycarbonyl L-phénylalanyl L-thréonyl L-séryl S-acétamidométhyl L-cystéine, p-phénylazobenzylsulfonyléthylester.

Acm
|
Boc—Phe—Thr—Ser—Cys—OPse

Acm
|
7,81 g (0,01 mole) de trifluoracétate de H—Thr—Ser—Cys—OPse

sont dissous dans 90 ml de DMF anhydre et traités à 0°C par 2,52 ml de N-éthylmorpholine, puis par 4,64 g de Boc—Phe—ONp.

Aprés une nuit au réfrigérateur, le produit est isolé par évaporation du DMF sous vide poussé à 30°C maximum, lavages du solide obtenu par l'eau, une solution bisulfate-sulfate de potassium, l'eau, le bicarbonate de sodium aqueux, l'eau et, après un léger séchage, par l'isopropanol et, enfin, à l'éther.

On obtient ainsi 8,22 g (90%); Fc 153—155°C.

CCM/EPAW$_1$ Rf = 0,84
EPAW$_2$ Rf = 0,67
BEW$_1$ Rf = 0,75
BPEW$_1$ Rf = 0,75

$[\alpha]_D^{25} = -16,0°$ (C = 1, $CH_3COOH$).

## Exemple 8

L-phénylalanyl L-thréonyl L-séryl S-acétamidométhyl L-cystéinate de p-phénylazobenzylsulfonyléthyle, trifluoracétate.

```
                  Acm
                   |
H—Phe—Thr—Ser—Cys—OPse, CF3COOH
```

```
                          Acm
                           |
9,14 g de  Boc—Phe—Thr—Ser—Cys—OPse
```

sont ajoutés à 55 ml d'acide trifluoracétique glacé sous agitation. On conserve ensuite pendant 5 à 10 min en laissant se réchauffer, puis évapore sous vide à 20—25°C. Le résidu est repris par beaucoup d'éther, trituré, filtré et lavé à l'éther.

On obtient ainsi 9,0 g (97%) d'un produit pur.

CCM/EPAW$_1$ Rf = 0,47
EPAW$_2$ Rf = 0,39
BPEW$_1$ Rf = 0,65
EBPEW Rf = 0,65
$[\alpha]_D^{25} = -18,5°$ (C = 1, $CH_3OH$).

## Exemple 9

N-t-butoxycarbonyl L-thréonyl L-phénylalanyl L-thréonyl L-séryl S-acétamidométhyl L-cystéine p-phénylazobenzylsulfonyléthylester.

```
                       Acm
                        |
Boc—Thr—Phe—Thr—Ser—Cys—OPse
```

```
                                            Acm
                                             |
9,28 g de trifluoracétate de  H—Phe—Thr—Ser—Cys—OPse
```

en solution dans 100 ml de DMF anhydre sont traités successivement au bain de glace par 2,52 ml de N-éthylmorpholine, puis par 3,80 g de Boc—Thr—ONSu. La solution est conservée une nuit au réfrigérateur. Le solvant est évaporé sous vide poussé à 30°C, le résidu repris par de la glace jusqu'à solidification, filtré, lavé à l'eau, à la solution aqueuse de sulfate-bisulfate de potassium, à l'eau, au bicarbonate de sodium aqueux, à l'eau, puis par 2 × 5 ml d'isopropanol et, enfin, à l'éther, et séché.

On obtient ainsi 9,1 g (90%) de produit. Sa pureté n'est généralement pas satisfaisante en CCM et il doit subir des lavages par les mélanges: isopropanol-eau: 50/50, puis 75/25, puis 100/0 (50 à 100 ml par lavage).

Rendement 86%.

Le produit pur présente les caractéristiques suivantes:

CCM/EPAW$_1$ Rf = 0,79
EPAW$_2$ Rf = 0,63
BEW$_1$ Rf = 0,66
TRUTH Rf = 0,05
Fc 169—172°C
$[\alpha]_D^{25} = -20°C$ (C = 1, AcOH).

## Exemple 10

L-thréonyl L-phénylalanyl L-thréonyl L-séryl S-acétamidométhyl L-cystéinate de p-phénylazobenzylsulfonyléthyle, trifluoracétate.

```
                                Acm
                                 |
CF3COOH, H—Thr—Phe—Thr—Ser—Cys—OPse
```

```
                               Acm
                                |
20,3 g de  Boc—Thr—Phe—Thr—Ser—Cys—OPse
```

sont dissous dans 160 ml de mélange T.F.A., chlorure de méthylène 50/50 (volume/volume) préalablement refroidis vers 5 à 10°C. Après 30 min à température ambiante la réaction est complète.

Les solvants sont évaporés sous vide et le résidu repris par l'isopropanol qui est évaporé. On reprend ensuite à l'éther anhydre, filtre, lave par beaucoup d'éther et sèche.

On obtient 20,3 g (98%).

CCM/$EPAW_1$ Rf = 0,50
$EPAW_2$ Rf = 0,33
EBPEW Rf = 0,69
$[\alpha]_D^{25} = -9,8°$ (C = 1, AcOH)

Exemple 11

N-α-t-butoxycarbonyl N-ω-méthylsulfonyléthoxycarbonyl L-lysyl L-thréonyl L-phénylalanyl L-thréonyl L-séryl S-acétamidométhyl L-cystéinate de p-phénylazobenzylsulfonyléthyle.

MSC Acm
| |
Boc—Lys—Thr—Phe—Thr—Ser—Cys—OPse

Acm
|
10,29 g du trifluoracétate du pentapeptide H—Thr—Phe—The—Ser—Cys—OPse

en solution dans 100 ml de DMF anhydre sont traités successivement à 0°C par 2,52 ml de N-éthylmorpholine, puis 6,2 g de

Acm
|
Boc—Lys—ONp.

La réaction est conservée à température ambiante pendant 15 à 20 h. L'isolement se fait alors de la manière habituelle: évaporation du solvant sous haut vide à 30°C, agitation en présence de glace, filtration, lavages abondants à l'eau, à la solution aqueuse de sulfate-bisulfate de potassium (pH 2), puis de nouveau à l'eau, suivis de deux lavages par 100 ml d'isopropanol et, enfin, abondamment à l'éther.

On obtient ainsi 11,4 g (88%) d'un produit qui, généralement, doit subir des lavages répétés à l'acétone (de 25 à 50 ml).

On obtient ainsi, avec un rendement de 80 à 85% un produit pur; Fc 172—174°C.

CCM/$EPAW_1$ Rf = 0,75
$EPAW_2$ Rf = 0,50
$BEW_1$ Rf = 0,57
$[\alpha]_D^{25} = -19,5°$ (C = 1, AcOH).

Exemple 12

N-ω-méthylsulfonyléthoxycarbonyl L-lysyl L-thréonyl L-phénylalanine L-thréonyl L-séryl S-acétamidométhyl L-cystéinate de p-phénylazobenzylsulfonyléthyle, trifluoracétate.

Msc Acm
| |
$CF_3COOH$, H—Lys—Thr—Phe—Thr—Ser—Cys—OPse

Msc Acm
| |
6,47 g (5 millimoles) de Boc—Lys—Thr—Phe—Thr—Ser—Cys—OPse

sont ajoutés à 52 ml d'acide trifluoracétique agités. Après dissolution totale (2 à 3 min), l'acide est évaporé sous vide à 20°C et le résidu repris par de l'isopropanol filtré sous vide, rincé deux fois par le même solvant puis à l'éther et séché en présence de potasse sous vide.

Le produit pèse 6,40 g (97%) et est pratiquement pur en

CCM/$EPAW_2$ Rf = 0,25
$EPAW_1$ Rf = 0,50.

Exemple 13

N-α-t-butoxycarbonyl L-tryptophanyl N-ω-méthylsulfonyléthoxycarbonyl L-lysyl L-thréonyl L-phénylalanyl L-thréonyl L-séryl S-acétamidométhyl L-cystéinate de p-phénylazobenzylsulfonyléthyle.

```
             Msc                          Acm
              |                            |
Boc—Trp—Lys—Thr—Phe—Thr—Ser—Cys—OPse
```

```
                                   Msc                          Acm
                                    |                            |
13,075 g de trifluoracétate de  H—Lys—Thr—Phe—Thr—Ser—Cys—OPse
```

sont mis en solution dans 130 ml de DMF anhydre et traités au bain de glace par 2,5 ml de N-éthylmorpholine, 5,11 g de Boc—Trp—ONp et 2,70 g d'hydroxybenzotriazole. Après 1 h environ à température ambiante, la réaction est complète, le solvant évaporé, le résidu repris par de la glace se solidifie peu à peu; il est finement broyé, filtré, lavé abondamment à l'eau puis aprés un léger séchage par 2 fois 120 ml d'isopropanol. tiède, puis par des fractions de 100 ml d'acétone tiède jusqu'à pureté satisfaisante en CCM (de 4 à 10 lavages selon les expériences). On obtient ainsi 12,6 g (85% du produit du titre présentant les caractéristiques suivantes:

CCM/$EAPW_2$ Rf ~ 0,50

$[\alpha]_D^{25} = -17,5°$ (C = 1, AcOH).

Exemple 14

N-α-biphénylisopropyloxycarbonyl L-tryptophanyl N-ω-méthylsulfonyléthoxycarbonyl L-lysyl L-thréonyl L-phénylalanyl L-thréonyl L-séryl S-acétamidométhyl L-cystéinate de p-phénylazobenzylsulfonyléthyle.

```
              Msc                          Acm
               |                            |
Bpoc—Trp—Lys—Thr—Phe—Thr—Ser—Cys—OPse
```

```
                                  Msc                          Acm
                                   |                            |
1,307 g de trifluoracétate de  H—Lys—Thr—Phe—Thr—Ser—Cys—OPse
```

en solution dans 13 ml de DMF anhydre sont traités à 0°C par 0,25 ml de N-éthylmorpholine et 0,666 g de Bpoc—Trp—ONp. Après un séjour de 48 h à +4°C ou 24 h à température ambiante la réaction est généralement terminée. Après évaporation du solvant sous haut vide à 30°C, le résidu se solidifie par agitation en présence de glace. Le précipité filtré, lavé par beaucoup d'eau puis par des solutions aqueuses de bicarbonate de sodium à 5% et de tampon citrate pH 3,5 et, enfin, par l'eau. Après séchage, il est lavé par trois fois 10 ml d'isopropanol tiède, puis trois fois 10 ml d'acétone tiède, ce qui généralement, suffit pour obtenir une pureté correcte.

On obtient ainsi, après séchage: 1,37 g (85%).

Ce produit est caractérisé par:

Fc 158—160°C.

$[\alpha]_D^{25} = -16,5°$ (C = 1, AcOH).

| | |
|---|---|
| CCM/$BEW_1$ | Rf = 0,70 |
| $EPAW_1$ | Rf = 0,76 |
| EBPEW | Rf = 0,77 |

Exemple 15

L-tryptophanyl N-ω-méthylsulfonyléthoxycarbonyl L-lysyl L-thréonyl L-phénylalanyl L-thréonyl L-séryl S-acétamidométhyl L-cystéinate de p-phénylazobenzylsulfonyléthyle, trifluoracétate.

```
                      Msc                          Acm
                       |                            |
CF3COOH, H—Trp—Lys—Thr—Phe—Thr—Ser—Cys—OPse
```

1,30 g de l'heptapeptide de l'exemple 14 est ajouté en une minute environ à un mélange préalablement refroidi à 5°C de 13 ml d'acide trifluoracétique, 1,3 ml d'éthanedithiol et 0,65 ml d'anisole. Après 2 min d'agitation à cette température, les réactifs volatils sont évaporés sous vide sans chauffer et le résidu repris par deux fois 15 ml de méthanol qui est évaporé à chaque fois sous vide. Le résidu trituré en présence de 15 ml d'isopropanol est filtré. On le lave ensuite par trois fois 15 ml du même solvant tiède (40°C environ) puis à l'éther et sèche.

On obtient ainsi 1,10 g (92%) du trifluoracétate qui se révèle pur en CCM dans les mélanges suivants:
$EPAW_1$ Rf = 0,36
$BEW_2$ Rf = 0,36

Exemple 16

α-Biphénylisopropyloxycarbonyl L-phénylalanyl L-tryptophanyl ω-méthylsulfonyléthoxycarbonyl L-lysyl L-thréonyl L-phénylalanyl L-thréonyl L-séryl S-acétamidométhyl L-cystéinate de p-phénylazobenzylsulfonyléthyle.

Msc Acm
| |
Bpoc—Phe—Trp—Lys—Thr—Phe—Thr—Ser—Cys—OPse

13,4 g du trifluoracétate de l'heptapeptide de l'exemple 15 en solution dans 130 ml de DMF sont traités successivement par 2,3 ml de N-éthylmorpholine, 2,43 g d'hydroxybenzotriazole et 4,25 g de Boc—Phe—ONp au bain de glace. Après 1 h de séjour à température ambiante, un contrôle CCM montre que la réaction est terminée. Le DMF est alors évaporé sous haut vide et le résidu agité en présence de glace se solidifie progressivement. Il est filtré, lavé, à l'eau et séché, puis lavé deux fois par l'isopropanol et trois fois par de l'acétone tiède (40°C).

On obtient ainsi 13,6 g (93%) d'un produit de pureté correcte en chromatographie.
CCM/$EPAW_1$ Rf = 0,77
$EPAW_2$ Rf = 0,58
$BPEW_1$ Rf = 0,69
$BEW_1$ Rf = 0,67
EBPEW Rf = 0,91
$[\alpha]_D^{25} = -9°$ (C = 1, DMF).

Exemple 17

Msc Acm
| |
H—Phe—Trp—Lys—Thr—Phe—Thr—Ser—Cys—OPse trifluoroacétate.

13,4 g (8,2 moles) de l'octapeptide de l'exemple 16 sont ajoutés à un mélange préalablement refroidi à 5°C de 130 ml TFA, 13 ml d'éthanedithiol et 6,5 ml d'anisole. L'agitation est poursuivie 15 min à température ambiante.

On évapore sous vide à température ambiante, puis on reprend le résidu deux fois par 70 ml de méthanol en évaporant à chaque fois le solvant. Puis on reprend le produit dans l'isopropanol et filtre le solide qu'on lave deux fois avec 70 ml d'isopropanol tiède (40°C). On lave abondamment avec de l'éther anhydre et sèche.

On obtient ainsi 13,1 g (97%) de produit pur.
CCM/$EPAW_1$ Rf = 0,38
$EPAW_2$ Rf = 0,32
$BEW_1$ Rf = 0,42
EBPEW Rf = 0,75

Exemple 18

Msc Acm
| |
Boc—Phe—Phe—Trp—Lys—Thr—Phe—Thr—Ser—Cys—OPse.

Acm Acm
| |
13,1 g de trifluoracétate de H—Phe—Trp—Lys—Thr—Phe—Thr—Ser—Cys—OPse

en solution dans 130 ml de DMF anhydre à 0°C sont traités successivement par 2 ml de N-éthylmorpholine, 2,16 g d'hydroxybenzotriazole et 3,70 g de Boc—Phe—ONp. L'agitation est poursuivie à température ambiante pendant 1 h. Le DMF est alors évaporé à 30°C sous vide et le résidu huileux cristallise peu à peu en présence de glace. Le précipité est filtré, lavé abondamment à l'eau et séché avant d'être finement broyé et lavé deux fois avec de l'isopropanol, trois fois avec du méthanol, puis cinq fois à l'acétone.

Après séchage, il pèse 13,5 g (95%).

CCM/EPAW$_2$ Rf = 0,62
BPEW$_1$ Rf = 0,70
EBPEW Rf = 0,94
$[\alpha]_D^{25} = -9,2°$ (C = 1, DMF).

Exemple 19

Msc Acm
| |
Bpoc—Phe—Phe—Trp—Lys—Thr—Phe—Thr—Ser—Cys—OPse.

Une solution de 0,98 g du trifluoracétate de

Msc Acm
| |
H—Phe—Trp—Lys—Thr—Phe—Thr—Ser—Cys—OPse

dans 12 ml de DMF est traitée à 0°C par 0,15 ml de N-éthylmorpholine et 0,378 g de Bpoc—Phe—ONp. La réaction est complétée après 24 h à température ambiante. Après évaporation du DMF à 30°C sous vide, le résidu est cristallisé en présence de glace, filtré sous vide, lavé successivement avec un tampon citrate pH 3,5 glacé, à l'eau, avec une solution saturée de bicarbonate de sodium, puis à l'eau et séché. Les lavages suivants, isopropanol trois fois 4 ml, acétone trois fois 3 ml et éther deux fois 5 ml, fournissent, après séchage, un produit qui est de nouveau lavé à l'acétone trois fois 4 ml.

On obtient finalement 1,02 g (89%) de produit pur.

CCM/EPAW$_1$ Rf = 0,82
EPAW$_2$ Rf = 0,58
$[\alpha]_D^{25} = -9,1°$ (C = 1, DMF).
F. 222—224°C (décomposition).

Exemple 20

Msc Acm
| |
TFA, H—Phe—Phe—Trp—Lys—Thr—Phe—Thr—Ser—Cys—OPse.

A température du laboratoire, on prépare le mélange suivant: TFA 300 ml, éthanedithiol 30 ml, anisole 15 ml. On additionne 30 g de

Msc Acm
| |
Bpoc—Phe—Phe—Trp—Lys—Thr—Phe—Thr—Ser—Cys—OPse,

garde 20 min à température du laboratoire, puis évapore à 30°C sous vide jusqu'à volume résiduel d'environ 70 ml.

Ce résidu est versé sur 700 ml de mélange propanol-2/éther éthylique 50/50 (volume/volume). Le précipité est essoré, lavé par le même mélange, puis par l'éther seul.

Après séchage, on obtient 30,2 g (100%).

CCM/EPAW$_2$ Rf = 0,40.

Exemple 21

Msc Acm
| |
Boc—Asn—Phe—Phe—Trp—Lys—Thr—Phe—Thr—Ser—Cys—OPse.

Msc Acm
| |
30,2 g de TFA, H—Phe—Phe—Trp—Lys—Thr—Phe—Thr—Ser—Cys—OPse

sont dissous dans 300 cm$^3$ de DMF puis traités successivement par 4,3 cm$^3$ de N-éthylmorpholine, 4,6 g d'hydroxybenzotriazole, 7,2 g de Boc—Asn—ONp. Après 30 min à température du laboratoire, la réaction est achevée. Le DMF est évaporé à 35°C sous vide jusqu'à un volume résiduel d'environ 100 ml. Cette solution est versée sous agitation sur un mélange eau-glace (500 ml), ce qui entraîne la

cristallisation du produit. Le solide est essoré, lavé à l'eau abondamment puis par le propanol-2 (deux fois), le méthanol (deux fois) et l'acétone (trois fois).

Après séchage, on obtient 28 g (87%).

CCM/EPAW$_2$ Rf = 0,70

$[\alpha]_D^{22} = -16°$ (C = 1, DMF).

Exemple 22

Msc (Lys), Acm (Cys)

TFA, H—Asn—Phe—Phe—Trp—Lys—Thr—Phe—Thr—Ser—Cys—OPse.

28,2 g du décapeptide de l'exemple 21 sont traités par TFA (280 ml), éthanedithiol (28 ml) et anisole (14 ml) pendant 20 min à température du laboratoire. L'évaporation du TFA sous vide à 30°C est menée jusqu'à un volume résiduel de 50—60 ml. On verse alors sur 500 ml de mélange propanol-2/éther 50/50 (volume/volume). On essore le précipité lave et sèche.

Après séchage, on obtient 28,5 g (100%).

CCM/EPAW$_2$ Rf = 0,60.

Exemple 23

Msc (Lys), Msc (Lys), Acm (Cys)

Boc—Lys—Asn—Phe—Phe—Trp—Lys—Thr—Phe—Thr—Ser—Cys—OPse.

Dans 310 ml de DMF anhydre, on dissout 30,5 g du trifluoracétate obtenu à l'exemple 22, puis on ajoute de la N-éthylmorpholine (4,05 ml), hydroxybenzotriazole (4,32 g) et

Msc (Lys)

Boc—Lys —ONp

(9,95 g). La réaction est terminée après 45 min à température ambiante. Le volume du DMF est réduit jusqu'à environ 100 ml par évaporation sous vide. La solution est versée sur 600—700 ml d'un mélange eau-glace. On essore le solide, lave à l'eau puis avec du méthanol (trois fois) et de l'acétone (trois fois).

Après séchage, on obtient 32 g (92%).

CCM/EPAW$_2$ Rf = 0,70

BEW$_1$ Rf = 0,50

$[\alpha]_D^{25} = -14,5°$ (C = 1, DMF).

Exemple 24

Msc (Lys), Msc (Lys), Acm (Cys)

TFA, H—Lys—Asn—Phe—Phe—Phe—Trp—Lys—Thr—Phe—Thr—Ser—Cys—OPse

Dans un mélange de dichlorométhane (150 ml), éthanedithiol (18 ml) et anisole (9 ml), on ajoute 30,3 g de l'undécapeptide de l'exemple 23. A la suspension ainsi obtenue, on ajoute, en une fois, 180 ml de TFA. Après 20 min à température ambiante, le TFA est évaporé sous vide à 30°C. L'évaporation est arrêtée lorsqu'il reste environ 60 ml dans le ballon. On verse alors sur 500 ml de mélange propanol-2/éther 50/50 (volume/volume). On essore le solide, lave et sèche et on obtient 30,5 g de solide (100%).

CCM/EPAW$_1$ Rf = 0,50.

## Exemple 25

```
           Acm  Msc                       Msc                     Acm
            |    |                         |                       |
     Boc—Cys—Lys—Asn—Phe—Phe—Trp—Lys—Thr—Phe—Thr—Ser—Cys—OPse
```

Dans 350 ml de DMF, on dissout 32,7 g de trifluoracétate obtenu à l'exemple 34. La solution est additionnée successivement de N-éthylmorpholine (3,8 ml), d'hydroxybenzotriazole (4,05 g) et de 7,44 g de

```
     Acm
      |
 Boc—Cys—ONp.
```

Après 45 min à température ambiante, la réaction est terminée. Le DMF est alors évaporé sous vide partiellement. Le volume résiduel (environ 100 ml) est versé sur un mélange eau-glace (600 ml). Le précipité est isolé par essorage, puis lavé par l'eau, le méthanol (trois fois) et l'acétone (trois fois).

Après séchage on obtient 32,8 g (93%).

CCM/EPAW$_2$ Rf = 0,75

BPEW$_1$ Rf = 0,65.

## Exemple 26

```
            Acm  Msc                       Msc                     Acm
             |    |                         |                       |
     TFA, H—Cys—Lys—Asn—Phe—Phe—Trp—Lys—Thr—Phe—Thr—Ser—Cys—OPse.
```

32,7 g du dodécapeptide de l'exemple 25 sont mis en suspension dans le mélange suivant: dichlorométhane 145 ml, éthanedithiol 17 ml, anisole 8 ml. On additionne ensuite en une seule fois 170 ml de TFA. Après 30 min à température du laboratoire, le TFA est évaporé sous vide à 30°C jusqu'à obtention d'un volume résiduel de 70 ml que l'on verse progressivement sur 700 ml de propanol-2. Le solide est essoré et lavé avec du propanol-2 et de l'éther.

Après séchage, on obtient 33 g (100%).

CCM/EPAW$_1$ Rf = 0,40.

## Exemple 27

```
                  Acm  Msc                       Msc                     Acm
                   |    |                         |                       |
     Boc—Ala—Gly—Cys—Lys—Asn—Phe—Phe—Trp—Lys—Thr—Phe—Thr—Ser—Cys—OPse.
```

On dissout 33 g du trifluoracétate de l'exemple 26 dans 330 ml de DMF, puis ajoute 3,6 ml de N-éthylmorpholine, 3,5 g d'hydroxybenzotriazole et 7,16 g de Boc—Ala—Gly—OTcp. La réaction est achevée au bout de 45 min à température ambiante. Le DMF est évaporé sous vide jusqu'à un volume résiduel d'environ 80 ml. On verse sur le mélange eau-glace (600 ml). On essore le solide, lave par l'eau, le méthanol (trois fois) et l'acétone (trois fois).

Après séchage, on obtient 32 g (93%).

CCM/EPAW$_2$ Rf = 0,70

BPEW$_1$ Rf = 0,60

$[\alpha]_D^{25} = -15°$ (C = 1, DMF).

## Exemple 28

```
                     Acm  Msc                       Msc                     Acm
                      |    |                         |                       |
     TFA, H—Ala—Gly—Cys—Lys—Asn—Phe—Phe—Trp—Lys—Thr—Phe—Thr—Ser—Cys—OPse.
```

On met en suspension 24,7 g du tétradécapeptide de l'exemple 27 dans 170 ml de dichlorométhane additionné de 20 ml d'éthanedithiol et 10 ml d'anisole. On ajoute 200 ml de TFA. Après 20 min à température ambiante, on évapore le TFA sous vide jusqu'à un volume résiduel de 60 ml environ. On verse le résidu sur du propanol-2 (600 ml). On essore, lave et sèche.

On obtient 24,8 g du produit (100%).

CCM/EPAW$_2$ Rf = 0,50.

Exemple 29

Di-Acm-3,14-dihydrosomatostatine

On dissout 57,5 g du trifluoracétate obtenu à l'exemple 28 dans 1 150 ml de DMF. On ajoute 115 ml de méthanol. On place au bain de glace puis, lorsque la température est inférieure à 5°C, on a commence l'addition d'une solution de baryte 0,2N (1 150 ml). L'addition a lieu en 40 min avec une température maximale de 15°C. Après la fin de l'addition de baryte, on agite sans bain de glace jusqu'à la température de ~—18°C. A ce moment, on fait barboter un courant de gaz carbonique dans le milieu pour atteindre pH 5—6. On centrifuge le carbonate de baryum. Le surnageant est évaporé sous vide à 35—40°C jusqu'à volume résiduel de 600 ml. Ce résidu est versé sous bonne agitation dans 4 000 ml d'acétate d'éthyle: un solide précipite. On agite pendant 1 h puis essore sur verre fritté, lave et sèche (à l'air puis au dessiccateur).

Rendement 42 g (100%) en produit brut.

La purification par chromatographic de partition sur colonne de Séphadex G 25 F ou par distribution à contre-courant permet d'obtenir un produit de très haute pureté, titrant plus de 98% en HPLC.

Le rendement est de 48%.

CCM/$BPEW_1$ Rf = 0,40.

Exemple 30

Dihydrosomatostatine

1,61 g de di-Acm-3,14-dihydrosomatostatine sont dissous dans 80 ml de AcOH pur et 72 ml d'eau distillée (mélange dégazé). A température du laboratoire, à l'obscurité, sous azote et sous agitation, on ajoute 8,2 ml de solution de nitrate d'argent 1N. On agite pendant 16 h. On évapore à sec, lave par l'eau distillée dégazée. On reprend dans le mélange DMF/eau (60/40) dégazé. On fait ensuite passer un courant d'hydrogène sulfuré, centrifuge le sulfur d'argent et évapore à sec le surnageant. On dissout le résidue dans 50 ml d'eau dégazée et lyophilise.

Rendement: 1,3 g (86%).

Le dosage du produit, soit par HPLC, soit par dosage des fonctions thiol, fait apparaître une pureté d'environ 70%.

Exemple 31

Somatostatine

Le sel d'argent obtenu à partir de 7 g de di-Acm-3,14-dihydrosomatostatine est transformé en 3,14-dihydrosomatostatine, selon la technique de l'exemple 30. La solution dans le diméthylformamide-eau obtenue après traitement à l'hydrogène sulfuré, centrifugation et dégazage est diluée dans 4,6 l d'une solution aqueuse d'acide acétique à 5% et ajoutée graduellement pendant 18 h à 5,6 l d'eau dégazée contenant 12 g de ferricyanure de potassium et 24 g d'acétate d'ammonium.

Le pH du milieu est maintenu pendant toute le durée de l'opération à 6,9 par addition de solution d'ammoniaque à 8%. Cette réaction doit être effectuée sous bonne agitation et sous atmosphère permanente de gaz inerte (azote).

Après la fin de la réaction, le pH du milieu est amené à 5 par addition d'acide acétique. On filtre la solution sur résine Biorad AG 3 × 4 afin d'éliminer les ions complexes, puis on fixe le produit sur une résine acide faible, type Biorex 70. On lave par une solution diluée (5%) d'acide acétique et on élue ensuite le produit par une solution d'acide concentrée (50%).

Après évaporation à sec de la solution sous vide poussé et sans chauffer, on obtient 5,2 g d'un produit brut de réaction.

Le produit est purifié sur colonne Séphadex G 25 (H=2 000 cm $\phi$ 50) en utilisant l'acide acétique N comme éluant. On obtient après lyophilisation 2,9 g d'un produit pulvérulent blanc titrant 98,4% en HPLC analytique.

Rendement: 46%; analyse d'acides $\alpha$-aminés: conforme. $[\alpha]_D^{25} = -39°$ (C = 1, eau) rapporté au produit exempt d'eau et d'acide acétique.

CCM/$BPEW_1$ Rf = 0,40.

Exemple 32

N-(o-nitrophénylsulfényl) L-asparaginyl L-phénylalanyl L-phénylalaninate de méthyle.

Nps—Asn—Phe—Phe—$OCH_3$

On dissout 80 g de H—Phe—Phe—$OCH_3$, $CF_3COOH$ dans 890 ml de DMF contenant 23 ml de N-éthylmorpholine. On ajoute alors 71,1 g de Nps—Asn—ONSu et 23 g d'hydroxybenzotriazole. On ajuste le pH à 7 par addition de N-éthylmorpholine et on agite une nuit à température ambiante.

On évapore à sec jusqu'à l'obtention d'une huile orange épaisse. On reprend le résidu dans l'éther, on triture et on obtient un solide jaune. On l'essore et on le lave à l'eau (deux fois 900 ml), puis au méthanol (deux fois 900 ml).

On essore, on lave à l'éther et le produit est séché à l'air.

Rendement: 88,50 g (82,1%).

CCM/éluant $BEW_1$
tache unique.

$[\alpha]_D^{25} = -33°$ (C = 1, DMF).

Exemple 33

N-(o-nitrophénylsulfényl) L-asparaginyl L-phénylalanyl L-phénylalanine.

Nps—Asn—Phe—Phe—OH

Dans un ballon muni d'une bonne agitation, on dissout presque totalement 139 g du tripeptide de l'exemple 32 dans 1 200 ml de pyridine, puis on ajoute 253 ml de soude N. L'agitation est poursuivie pendant 30 min à température ambiante.

Un solide commence à précipiter, on ajoute 2 430 ml d'éther pour terminer la précipitation. Le solide jaune formé est essoré, puis repris dans 3 800 ml d'eau et acidifié en une seule fois par 1 300 ml d'acide acétique glacial. Le solide obtenu est essoré, puis lavé à l'eau et essoré. On effectue un deuxième lavage avec du méthanol.

Après essorage, le produit est séché.

Rendement: 127,46 g (94%).

CCM/éluant $BPEW_2$
une tache.

$[\alpha]_D^{25} = -22°$ (C = 1, DMF).

Exemple 34

L-asparaginyl L-phénylalanyl L-phénylalanine, chlorhydrate.

H—Asn—Phe—Phe—OH, HCl

On dissout 150 g de Nps—Asn—Phe—Phe—OH dans 1,32 l de DMF. On ajoute ensuite 2,5 équivalents d'une solution HCl gaz dans le méthanol anhydre (283 $cm^3$ de solution 2,28 N) en maintenant la température à 20°C. Le milieu est agité pendant 30 min, puis évaporé à sec à 30°C/0,5 mm Hg.

Le résidu pâteaux est trituré dans l'acétate d'éthyle jusqu'à l'obtention d'un solide. Le solide est essoré et lavé plusieurs fois à l'éther et séché.

Rendement: 118,38 g (98%).

CCM/éluant $BPEW_1$
une tache.

$[\alpha]_D^{25} = -9,5°$ (C = 1, DMF).

Exemple 35

N-$\alpha$-tertiobutoxycarbonyl N-$\omega$-méthylsulfonyléthoxycarbonyl L-lysyl L-asparaginyl L-phénylalanyl L-phénylalanine.

Msc
|
Boc—Lys—Asn—Phe—Phe—OH

On dissout 54,72 g de H—Asn—Phe—Phe—OH, HCl dans 354 ml de DMF contenant 59 ml d'eau. On ajoute 30 ml de N-éthylmorpholine puis 63 g de

Msc
|
Boc—Lys—ONp

en solution dans 236 ml de DMF et 16,15 g d'hydroxybenzotriazole. On ramène le pH à 7,7 par addition de N-éthylmorpholine et on laisse agiter pendant 2 h en maintenant le pH à 7,7.

Au bout de ces 2 h, on rajoute 6,3 g de

Msc
|
Boc—Lys—ONp

(10% d'excès) et on poursuit l'agitation pendant une nuit à température ambiante. On évapore le mélange à sec (30°C/0,5 mm Hg), on reprend le résidu par 600 ml d'eau presque saturés en ClNa et

600 ml d'acétate d'éthyle. On amène le pH à 4—4,5 par AcOH, décante et réextrait par 600 ml d'acétate d'éthyle. Les phases acétate d'éthyle sont réunies et additionnées de deux volumes d'éther.

Un précipité se forme que l'on abandonne quelques heures au réfrigérateur. Le solide est essoré, lavé avec le minimum d'acétate d'éthyle, puis abondamment à l'éther. Le produit obtenu est ensuite lavé avec 1,2 l de solution sulfate-bisulfate de potassium pendant 30 min. Il est essoré, bien lavé à l'eau, essoré et repris dans l'éther, puis essoré et séché.

Rendement 85g (95%).

CCM/éluant $BEW_1$

une tache.

$[\alpha]_D^{25} = -26{,}5°$ (C = 1, DMF).

Exemple 36

N-ω-méthylsulfonyléthoxycarbonyl L-lysyl L-asparginyl L-phénylalanyl L-phénylalanine, trifluoracétate.

```
  Msc
   |
H—Lys—Asn—Phe—Phe—OH, CF3CO2H
```

On dissout 82 g du peptide de l'exemple 35 dans 400 ml de TFA préalablement refroidi à +5°C et continue l'agitation 15 min à température ambiante. On filtre l'insoluble s'il y a lieu.

On évapore (30°C/0,1 mm) jusqu'à obtention d'une huile. On reprend cette huile par 1,6 l d'isopropanol et 100 ml d'acétate d'éthyle. Il en résulte une précipitation immédiate.

On essore, lave à l'éther et sèche.

Rendement: 76,82 g (92%).

CCM/éluant $BEW_1$

une tache.

$[\alpha]_D^{25} = -5{,}1°$ (C = 1, DMF).

Exemple 37

N-tertiobutoxycarbonyl S-acétamidométhyl L-cystéinyl N-ω-méthylsulfonyléthoxycarbonyl L-lysyl L-asparaginyl L-phénylalanyl L-phénylalanine.

```
     Acm  Msc
      |    |
Boc—Cys—Lys—Asn—Phe—Phe—OH
```

On dissout 106 g du trifluoracétate de l'exemple 36 dans 700 ml de DMF contenant 116 ml d'eau et 32,40 ml de N-éthylmorpholine. On ajoute ensuite 54,64 g de

```
     Acm
      |
Boc—Cys—ONp
```

dissous dans 466 ml de DMF et 18 g d'hydroxybenzotriazole. On ramène à pH 7,7 par addition de N-éthylmorpholine et on agite pendant 2 h à température ambiante en maintenant le pH à cette valeur.

Au bout de ces 2 h, on rajoute 5,46 g de

```
     Acm
      |
Boc—Cys—ONp
```

(10% d'excès) et on poursuit l'agitation à température ambiante pendant une nuit. On évapore à sec sous vide (30°C/0,5 mm Hg) et le gel obtenu est repris par 1 200 ml d'eau à moitié saturée de chlorure de sodium et 1 200 ml d'acétate d'éthyle. Si le pH est trop bas, on le ramène vers 6,5 par de la N-éthylmorpholine, on agite et on amène le pH à 4—4,5 par addition d'acide acétique.

Un précipité se forme, on laisse sous agitation 1 h dans la glace, puis on abandonne au repos 1 h et on essore.

On reprend le résidu dans un peu d'eau, on le triture, on essore, puis on le reprend dans 2,4 l de solution de sulfate-bisulfate de potassium et on agite 2 h. On essore, on lave à l'eau, puis on le fait sécher à l'air. On reprend le solide par le minimum d'acétone (700 ml), on le triture et on rejoute 4 à 5 volumes d'éther. On essore le précipité, lave à l'éther et sèche.

Rendement: 123,15 g (96,3%).
CCM/éluant $BEW_1$
tache unique
$[\alpha]_D^{25} = -28,6°$ (C = 1, DMF).

Exemple 38

S-acétamidométhyl L-cystéinyl N-ω-méthylsulfonyléthoxycarbonyl L-lysyl L-asparaginyl L-phénylalanyl L-phénylalanine, trifluoracétate.

$$H—\underset{}{\overset{Acm}{Cys}}—\overset{Msc}{Lys}—Asn—Phe—Phe—OH,\ CF_3CO_2H$$

On ajoute 86,44 g du peptide de l'exemple 37 à un mélange de 346 ml d'acide trifluoracétique et 34,6 ml d'éthanedithiol refroidi préalablement à 0°C.

Le solide se dissout en 15 min environ et la solution est conservée 15 min à température ambiante sous agitation. On évapore à sec (30°C/0,1 mm Hg), puis on reprend dans l'acétate d'éthyle et évapore. On répète deux fois ce traitement puis on reprend dans l'éther et essore. On lave abondamment à l'éther et sèche.

Poudre blanche.
Rendement: 85,96 g (98%).
CCM/éluant $BEW_1$
une tache
$[\alpha]_D^{25} = -14,4°$ (C = 1, DMF).

Exemple 39

N-tertiobutoxycarbonyl L-alanylglycyl S-acétamidométhyl L-cystéinyl N-ω-méthylsulfonyléthoxycarbonyl L-asparaginyl L-phénylalanyl L-phénylalanine.

$$Boc—Ala—Gly—\overset{Acm}{Cys}—\overset{Msc}{Lys}—Asn—Phe—Phe—OH$$

On dissout 119 g du trifluoracétate de l'exemple 38 dans 780 ml de diméthylformamide contenant 130 ml d'eau, puis on ajoute 30,3 ml de N-éthylmorpholine et, aussitôt, 51,1 g de Boc—Ala—Gly—OTcp en solution dans 518 ml de diméthylformamide.

On ajoute ensuite 16,21 g d'hydroxybenzotriazole sec et poursuit l'agitation à température ambiante pendant 2 h, après avoir ajusté le pH à 7,7 par la N-éthylmorpholine.

On ajoute ensuite 5,11 g de Boc—Ala—Gly—OTcp solide. On ajuste à nouveau le pH à 7,7 par la N-éthylmorpholine et laisse agiter une nuit à température ambiante.

On évapore à sec (30°C/0,1 mm Hg). L'huile obtenu est reprise par un mélange de 1 litre d'acétate d'éthyle et 1 litre d'eau presque saturée en chlorure de sodium. On porte le pH à 5—5,5 par de l'acide acétique et laisse agiter vigoureusement dans un bain de glace pendant 1 h.

On essore le solide, lave le gâteau par un peu de mélange acétate d'éthyle-eau presque saturée de chlorure de sodium. On triture ensuite le solide dans le minimum d'eau, puis on essore le solide et agite pendant 2 h avec une solution tamponnée à pH 2 (solution sulfate-bisulfate de potassium). On essore, lave à l'eau et sèche une nuit à l'air. On lave ensuite abondamment à l'ether.

On obtient une poudre blanche.
Rendement: 129,1 g (98%).
CCM/éluants $BPEW_1$
$EPAW_1$
une tache.
$[\alpha]_D^{25} = -27,3°$ (C = 1, DMF).

Exemple 40

$$Boc—Ala—Gly—\overset{Acm}{Cys}—\overset{Msc}{Lys}—Asn—Phe—Phe—Trp—\overset{Msc}{Lys}—Thr—Phe—Thr—Ser—\overset{Acm}{Cys}—OPse.$$

On dissout 75,5 g du trifluoracétate de l'heptapeptide de l'exemple 15 dans 1 150 ml de diméthylformamide et ajoute à la solution 6,3 ml de N-éthylmorpholine. Par ailleurs, on dissout 58,13 g de l'heptapeptide de l'exemple 39 dans 1 430 ml de diméthylformamide et ajoute à cette solution 10,81 g de dicyclohexylcarbodiimide et 14,18 g d'hydroxybenzotriazole sec.

On ajoute la première solution dans la deuxième et maintient le pH à 7 par addition de N-éthylmorpholine. On laisse sous agitation à température ambiante toute la nuit.

On verse le mélange réactionnel dans deux volumes d'éther. On laisse sous agitation 10 min, puis 10 min au repos à température ambiante. On essore le précipité obtenu et lave abondamment à l'éther.

Le solide ainsi obtenu est mis sous agitation vigoureuse pendant 1 h dans 1 litre d'éther. On essore et sèche.

On obtient un solide jaune.

Rendement: 100 g (81%).

Ce produit est en tout point identique à celui obtenu dans l'exemple 27.

CONTROLES ANALYTIQUES

Ainsi qu'il a été dit précédemment les purifications poussées n'interviennent qu'à deux stades du procédé de synthèse selon l'invention, à savoir le di-Acm-3,14-dihydrosomatostatine et la somatostatine elle-même.

Par suite, dans toutes les autres étapes, les contrôles analytiques sont limités à des chromatographies sur couche mince de silice dans divers systèmes solvants, ainsi qu'il est indiqué dans les exemples.

Par contre, pour les deux étapes de purification poussée, les contrôles analytiques sont beaucoup plus importants. Aux méthodes usuelles en chimie peptidique, telles que l'analyse d'acides aminés, vient s'ajouter une méthode de contrôle par HPLC.

L'appareil utilisé est composé d'un chromatographe liquide "Waters Associates": injecteur universel modèle U 6 K, pompe modèle 6000 A, détecteur multilongueur d'onde modèle 450, enregistreur Sefram modèle Servotrace.

Les conditions de chromatographie sont les suivantes:

Colonne: colonne 300 × 3,9 mm remplie du support $\mu$ Bondapak $C_{18}$ 10 $\mu$m (Waters Associates).

Solvant d'élution: le solvant d'élution est composé de mélange:

— solution aqueuse d'acétate d'ammonium $10^{-2}$M tamponnée à pH 4 par de l'acide acétique: 700 ml,

— acétronitrile pour spectroscopie: 250 ml.

Débit: 4 ml/min.

Pression: 210 bars.

Détecteur: détecteur UV à 210 nm.

Sensibilité 0,2 D.O. pleine échelle.

Enregistreur: 10 mV pleine échelle, déroulement 1 cm/min.

Les chromatogrammes obtenus avec le di-Acm-3,14-dihydrosomatostatine et la somatostatine sont représentés, respectivement, dans les figures 1 et 2.

Ils montrent clairement l'unicité du produit après les purifications mentionnées dans les exemples correspondants.

**Revendications**

1. Procédé de préparation de la somatostatine de formule I:

H— Ala—Gly—Cys—Lys—Asn—Phe—Phe—Trp—Lys—Thr—Phe—Thr—Ser—Cys—OH,
1 2 3 4 5 6 7 8 9 10 11 12 13 14

par élongation progressive de la chaîne peptidique à partir de la cystéine en position 14, en couplant successivement chaque aminoacide ou peptide dont les groupes aminés et thiol sont préalablement protégés, le groupe $\alpha$-aminé étant libéré et activé sous forme d'ester avant la réaction de formation de la liaison peptidique suivante, caractérisé en ce que la fonction acide de la cystéine de départ est protégée par le groupe phénylazobenzylsulfonyléthyloxy (OPse) qui rend tous les peptides intermédiaires insolubles dans l'eau et les solvants organiques usuels à l'exception du diméthylformamide (DMF) et du diméthylsulfoxyde (DMSO) et permet d'opérer toutes les réactions de couplage peptidique en solution dans le DMF ou le DMSO, de précipiter les peptides intermédiaires par addition d'un deuxième solvant et de purifier ces peptides par lavage en phase solide à l'aide de solvants appropriés qui dissolvent les impuretés.

2. Procédé selon la revendication 1, caractérisé en ce que les groupes thiol de la cystéine de départ et de la cystéine en position 3 sont protégés avant la réaction de couplage par un groupe acétamidométhyle (Acm) qui est conservé jusqu'à la dernière étape d'élongation de la chaîne peptidique.

3. Procédé selon l'une des revendications 1 et 2, caractérisé en ce que le groupe $\omega$-aminé de la lysine est protégé par un groupe méthanesulfonyléthyloxycarbonyl (Msc) avant le couplage de cet aminoacide en position 9 et en position 4, groupe résistant aux traitements acides de libération des

groupes $\alpha$-aminés temporairement protégés, et étant ainsi conservé jusqu'à la dernière étape d'élongation de la chaîne peptidique.

4. Procédé selon l'une des revendications 1 à 3 caractérisé en ce que la protection temporaire des groupes $\alpha$-aminés est assurée par un groupe N-tertiobutoxycarbonyle (Boc) ou par un groupe N-$\alpha$-biphénylisopropyloxycarbonyle (BPoc) qui est éliminé, avant la réaction suivante de couplage, par un acide fort tel que l'acide trifluoracétique ou l'acide formique à 1% d'acide chlorhydrique.

5. Procédé selon l'une des revendications 1 à 4, caractérisé en ce que le noyau indole du L-tryptophane, après introduction de cet aminoacide en position 8, est protégé par addition, dans le milieu, d'anisole et de thiols tels que l'éthanedithiol ou le mercaptoéthanol.

6. Procédé selon l'une des revendications 1 à 5, caractérisé en ce que les réactions de couplage sont activées par l'addition d'hydroxybenzotriazole.

7. Procédé selon la revendication 1, caractérisé en ce que, après avoir formé l'heptapeptide 8 à 14 protégé de formule III:

Msc (on Lys), Acm (on Cys)

H—Trp—Lys—Thr—Phe—Thr—Ser—Cys—OPse

on effectue son couplage directement avec l'heptapeptide 1 à 7 protégé de formule IV:

Acm (on Cys), Msc (on Lys)

Boc—Ala—Gly—Cys—Lys—Asn—Phe—Phe—OH

préalablement formé.

8. Procédé selon la revendication 7, caractérisé en ce que la réaction de couplage entre les deux heptapeptides protégés est effectuée dans le DMF, en présence d'un agent de couplage tel que le dicyclohexylcarbodiimide ou d'un activateur de la fonction carboxylique tel que l'hexafluorophosphate de benzotriazolyloxy(tridiméthylamino)phosphonium.

9. Procédé selon l'une des revendications 1 à 8, caractérisé en ce que le tétradécapeptide protégé obtenu, après la dernière réaction de couplage, de formule V:

Acm (on Cys), Msc (on Lys), Msc (on Lys), Acm (on Cys)

Boc—Ala—Gly—Cys—Lys—Asn—Phe—Phe—Trp—Lys—Thr—Phe—Thr—Ser—Cys—OPse

est a) traité par un acide fort tel que l'acide trifluoroacétique qui libère la fonction amine de l'alanine en position 1, puis b) traité par une base forte telle que la baryte pendant 15 à 30 min et à température ambiante, en solution dans le DMF, ce qui éliminte les groupes protecteurs des chaînes latérales des 2 molécules de lysine et l'ester sur la cystéine en position 14, pour donner la di-S-acétamidométhyl-3,14-dihydrosomatostatine qui, après purification, est c) traitée de façon connue en soi pour libérer les fonctions thiol des deux molécules de cystéine et donner la dihydrosomatostatine, laquelle est d) soumise au pontage final en disulfure entre les deux molécules de cystéine, par un procédé connu en soi, pour donner la somatostatine.

**Patentansprüche**

1. Verfahren zur Herstellung von Somatostatin der Formel I:

H—Ala—Gly—Cys—Lys—Asn—Phe—Phe—Trp—Lys—Thr—Phe—Thr—Ser—Cys—OH (Cys 3 and Cys 14 joined by a bridge)

1 2 3 4 5 6 7 8 9 10 11 12 13 14

durch progressive Verlängerung der Peptidkette ausgehend von Cystein in Position 14 durch sukzessive Kupplung jeder Aminosäure oder jedes Peptids, deren Amino- und Thiolgruppen zunächst geschützt sind, wobei vor der Reaktion zur Bildung der folgenden Peptidbindung die $\alpha$-Aminogruppe freigesetzt wird und Aktivesterbildung erfolgt, dadurch gekennzeichnet, daß die Säurefunktion des Ausgangscysteins durch die Phenylazobenzylsulfonyläthyloxy-(OPse)-Gruppe geschützt wird, welche sämtliche Zwischenpeptide unlöslich in Wasser und den gebräuchlichen organischen Lösungsmitteln, mit Ausnahme von Dimethylformamide (DMF) und Dimethylsulfoxid (DMSO), macht und die Durchführung sämtlicher Peptidkupplungsreaktionen in Lösung in DMF oder DMSO, die Ausfällung der Zwischenpeptide durch Zugabe eines zweiten Lösungsmittels und die Reinigung dieser Peptide durch Waschen mit geeigneten, die Verunreinigungen lösenden Lösungsmitteln in fester Phase gestattet.

2. Verfahren nach Anspruch 1, dadurch gekennzeichnet, daß die Thiolgruppen des Ausgangscysteins und des Cysteins in Position 3 vor der Kupplungsreaktion durch eine Acetamidomethyl-(Acm)-Gruppe geschützt werden, welche bis zum letzten Verlängerungsschritt der Peptidkette konserviert wird.

3. Verfahren nach einem der Ansprüche 1 und 2, dadurch gekennzeichnet, daß die $\omega$-Aminogruppe von Lysin vor der Kupplung dieser Aminosäure in Position 9 und Position 4 durch eine Methansulfonyläthyloxycarbonyl-(Msc)-Gruppe geschützt wird, welche gegen die Säurebehandlungen zur Freisetzung der zeitweise geschützten $\alpha$-Aminogruppen beständig ist und so bis zum letzten Verlängerungsschritt der Peptidkette konserviert wird.

4. Verfahren nach einem der Ansprüche 1 bis 3, dadurch gekennzeichnet, daß der zeitweise Schutz der $\alpha$-Aminogruppen durch eine N-tert. Butoxycarbonyl-(Boc)-Gruppe oder durch eine N-$\alpha$-Biphenylisopropyloxycarbonyl-(BPoc)-Gruppe bewerkstelligt wird, welche vor der folgenden Kupplungsreaktion mittels einer starken Säure, wie Trifluoressigsäure oder Ameisensäure mit 1% Salzsäure entfernt wird.

5. Verfahren nach einem der Ansprüche 1 bis 4, dadurch gekennzeichnet, daß der Indolkern des L-Tryptophans nach Einführung dieser Aminosäure in Position 8 durch Zugabe von Anisol und von Thiolen, wie Äthandithiol oder Mercaptoäthanol, in das Medium geschützt wird.

6. Verfahren nach einem der Ansprüche 1 bis 5, dadurch gekennzeichnet, daß die Kupplungsreaktionen durch Zugabe von Hydroxybenzotriazol aktiviert werden.

7. Verfahren nach Anspruch 1, dadurch gekennzeichnet, daß nach Bildung des geschützten Heptapeptids 8 bis 14 der Formel III:

```
            Msc                         Acm
             |                           |
H—Trp—Lys—Thr—Phe—Thr—Ser—Cys—OPse
```

dessen direkte Kupplung mit dem vorher gebildeten geschützten Heptapeptid 1 bis 7 der Formel IV:

```
                 Acm  Msc
                  |    |
Boc—Ala—Gly—Cys—Lys—Asn—Phe—Phe—OH
```

durchgeführt wird.

8. Verfahren nach Anspruch 7, dadurch gekennzeichnet, daß die Kupplungsreaktion zwischen den beiden geschützten Heptapeptiden in DMF in Anwesenheit eines Kupplungsmittels, wie Dicyclohexylcarbodiimid oder eines Aktivators der Carboxylfunktion, wie Benzotriazolyloxy-(tridimethylamino)-phosphonium-Hexafluorphosphat, durchgeführt wird.

9. Verfahren nach einem der Ansprüche 1 bis 8, dadurch gekennzeichnet, daß das nach der letzten Kupplungsreaktion erhaltene geschützte Tetradecapeptid der Formel V

```
             Acm  Msc                   Msc                   Acm
              |    |                     |                     |
Boc—Ala—Gly—Cys— Lys—Asn—Phe—Phe—Trp—Lys—Thr—Phe—Thr—Ser—Cys—OPse
```

a) mit einer starken Säure, wie Trifluoressigsäure, behandelt wird, welche die Aminofunktion des Alanins in Position 1 freisetzt, danach b) mit einer starken Base, wie Barytlauge, während 15 bis 30 min und bei Umgebungstemperatur in Lösung in DMF behandelt wird, wodurch die Schutzgruppen der Seitenketten der 2 Moleküle des Lysins und die Estergruppe am Cystein in Position 14 unter Entstehung von di-S-Acetamidomethyl-3,14-dihydrosomatostatin entfernt werden, welches nach Reinigung c) auf an sich bekannte Weise behandelt wird, um die Thiolfunktionen der beiden Cysteinmoleküle freizusetzen und Dihydrosomatostatin zu ergeben, welches d) einer abschließenden Disulfidbrückenbildung zwischen den beiden Cysteinmolekülen nach einem an sich bekannten Verfahen unterworfen wird, um Somatostatin zu ergeben.

## Claims

1. Process for the preparation of somatostatin of formula I:

```
            ┌──────────────────────────────────────────────┐
H— Ala—Gly—Cys—Lys—Asn—Phe—Phe—Trp—Lys—Thr—Phe—Thr—Ser—Cys—OH,
    1   2   3   4   5   6   7   8   9   10  11  12  13  14
```

by progressive elongation of the peptide chain from the cysteine in 14 position, by successively coupling each aminoacid or peptide whose amino and thiol groups are previously protected, the $\alpha$-amino group being released and activated in the form of ester before the reaction of formation of the following peptide bond, characterized in that the acid function of the starting cysteine is protected by the phenylazobenzylsulfonylethyloxy (OPse) group which renders all the intermediate peptides insoluble in water

and the usual organic solvents with the exception of dimethylformamide (DMF) and dimethylsufoxide (DMSO) and makes it possible to effect all the peptide coupling reactions in solution in the DMF or DMSO, to precipitate the intermediate peptides by addition of a second solvent and to purify these peptides by washing in solid phase with the aid of suitable solvents which dissolve the impurities.

2. A process according to claim 1, characterized in that the thiol groups of the starting cysteine and the cysteine in 3 position are protected before the coupling reaction by an acetamidomethyl (Acm) group which is conserved up to the last stage of elongation of the peptide chain.

3. A process according to any one of claims 1 and 2, characterized in that the $\omega$-amino group of the lysine is protected by a methanesulfonylethyloxycarbonyl (Msc) group before the coupling of this aminoacid in 9 position and in 4 position, group resisting the acid treatments of release of the temporarily protected $\alpha$-amino groups, and thus being conserved up to the last stage of elongation of the peptide chain.

4. A process according to any one of claims 1 to 3, characterized in that the temporary protection of the $\alpha$-amino groups is ensured by an N-tertiobutoxycarbonyl group (Boc) or by an N-$\alpha$-biphenyl-isopropyloxycarbonyl (BPoc) group which is eliminated, before the following coupling reaction, by a strong acid such as trifluoroacetic acid or formic acid with 1% hydrochloric acid.

5. A process according to any one of claims 1 to 4, characterized in that the indole nucleus of the L-tryptophane, after introduction of this aminoacid in 8 position, is protected by addition, in the medium, of anisole and of thiols such as ethanedithiol or mercaptoethanol.

6. A process according to any one of claims 1 to 5, characterized in that the coupling reactions are activated by the addition of hydroxybenzotriazol.

7. A process according to claim 1, characterized in that, after having formed the protected heptapeptide 8 to 14 of formula III:

—H—Trp—Lys(Msc)—Thr—Phe—Thr—Ser—Cys(Acm)—OPse

its coupling is effected directly with the protected heptapeptide 1 to 7 of formula IV

Boc—Ala—Gly—Cys(Acm)—Lys(Msc)—Asn—Phe—Phe—OH

previously formed.

8. A process according to claim 7, characterized in that the coupling reaction between the two protected heptapeptides is effected in DMF, in the presence of a coupling agent such as dicyclohexylcarbodiimide or of an activator of the carboxylic function such as the hexafluorophosphate of benzotriazolyloxy (tridimethylamino)phosphonium.

9. A process according to any one of claims 1 to 8, characterized in that the protected tetradecapeptide obtained, after the last coupling reaction of formula V:

Boc—Ala—Gly—Cys(Acm)—Lys(Msc)—Asn—Phe—Phe—Trp—Lys(Msc)—Thr—Phe—Thr—Ser—Cys(Acm)—OPse

is a) treated with a strong acid such as trifluoroacetic acid which releases the amine function of the alanine in 1 position, then b) treated with a strong base such as baryte for 15 to 30 mins. and at ambient temperature, in solution in DMF, this eliminating the protector groups of the side chains of the 2 molecules of lysine and the ester on the cysteine in 14 position, to give di-S-acetamido-methyl-3,14-dihydrosomatostatin which, after purification, is c) treated in manner known per se to release the thiol functions of the two molecules of cysteine and give the dihydrosomatostatin, which is d) subject to final bridging in disulfide between the two cysteine molecules, by a process known per se, to give somatostatin.

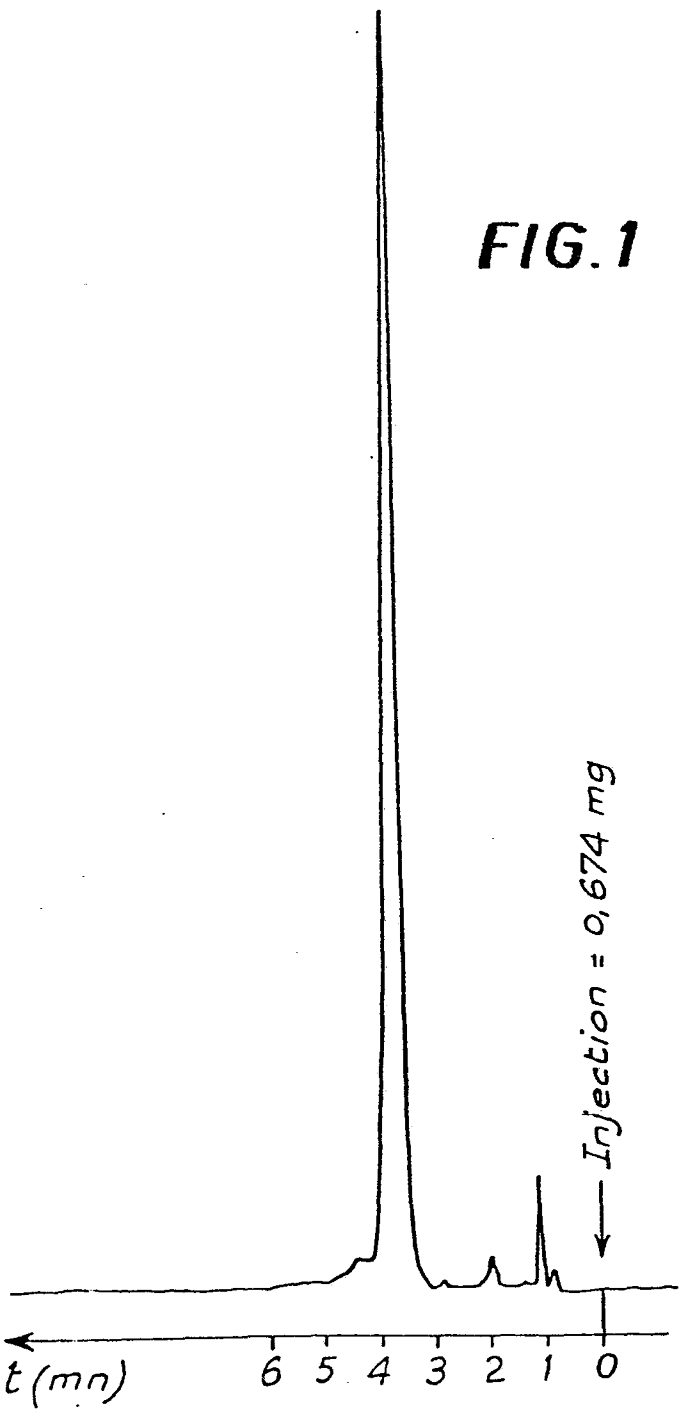
Injection = 0,674 mg
t (mn)
6 5 4 3 2 1 0

FIG.1

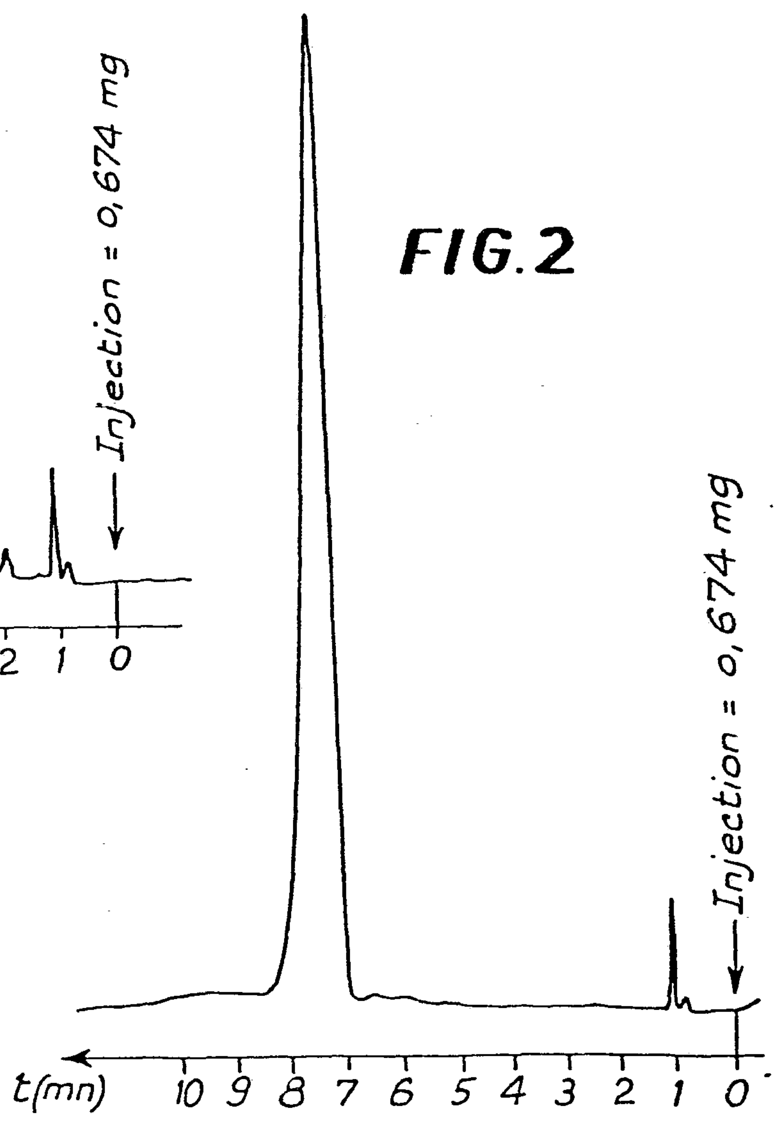
Injection = 0,674 mg
t(mn)
10 9 8 7 6 5 4 3 2 1 0

FIG.2